# EUROPEAN PATENT SPECIFICATION

(11) **EP 3 996 643 B1**
(45) Date of publication and mention of the grant of the patent: **05.08.2026**
(21) Application number: 20739365.3
(22) Date of filing: 10.07.2020
(51) Int. Cl.: A61F 7/12, A61B 1/303, A61H 19/00, A61H 9/00, A61B 17/34, A61B 90/00, A61B 90/90, A61B 17/42

(54) **A DEVICE FOR TREATING VAGINAL ATROPHY**
VORRICHTUNG ZUR BEHANDLUNG VON VAGINALER ATROPHIE
DISPOSITIF POUR LE TRAITEMENT DE L'ATROPHIE VAGINALE

(30) Priority: 11.07.2019 EP 19185897
(43) Date of publication of application: 18.05.2022
(73) Proprietor: University of Galway, Galway (IE)
(72) Inventor: NEWELL, Paula, Galway (IE); EATON-EVANS, Jimmy, Galway (IE)
(74) Representative: Barker Brettell LLP
(86) International application number: PCT/EP2020/069668
(87) International publication number: WO 2021/005240

(56) References cited:
- US-A- 4 141 360
- US-A1- 2008 154 183
- US-A1- 2009 065 565
- US-A1- 2009 281 482
- US-A1- 2010 274 260
- US-A1- 2016 242 953
- US-A1- 2016 317 078
- US-A1- 2017 035 949
- US-A1- 2018 296 383

## Description

### Field of the Invention

The present invention relates to a device for treating vaginal atrophy. Also contemplated, but not part of the claimed subject-matter, are methods of treating vaginal atrophy/dryness.

### Background to the Invention

Vulva and/or Vaginal Atrophy (VVA / VA) is a chronic & progressive condition because of a reduction or absence of oestrogen. Vaginal Atrophy (VA) is a chronic and progressive condition that affects approximately 80% of women because of hormonal changes at some point during their lifetime. It results from reduced oestrogen levels that cause the vagina and surrounding tissue to become dry, thin and inflamed. Symptoms include chronic dryness, itching, irritation and pain. Studies have shown it impairs a woman's quality of life, affects intimate relationships and makes it uncomfortable to sit, stand, exercise, urinate or even work. VA is a prevalent condition and is most common in women in the menopausal age range and Breast Cancer Survivors (BCSs). It is estimated that by 2020, there will be 50 million menopausal women in the US and by 2025 there will be 1.1 billion women in the menopausal range across the world suffering from VA. Additionally, there are 3.1 million BCSs suffering with VA. There is currently no affordable, safe, non-hormonal and effective treatment for VA. The present disclosure seeks to address this clinical need.

When a woman experiences the menopause, oestrogen production is decreased by 70%. With VA, the tissues of a woman's vagina no longer work in their normal, healthy way. The lining of the vagina begins to shrink, and walls become thinner and drier that makes for an uncomfortable dry sensation that affects a woman's quality of life. The vaginal epithelium becomes thinner, elasticity is reduced, vaginal secretions decrease and blood flow is reduced.

More than 70% of BCSs become postmenopausal after chemotherapy and radiation treatment and consequently BCSs experience VA. The chemotherapy and radiation cause a reduction in oestrogen hence inducing early menopause. Additionally, the cancer blocker and endocrine drugs that women take to remain cancer free, further exacerbate the symptoms of VA owing to the oestrogen suppression effect of these therapies that worsens the symptoms of itchiness, pain and dryness around the vagina area. Additionally, VA is often the most poorly addressed side effect for BCSs on adjuvant endocrine therapy. It is an object of the invention to overcome at least one of the above-referenced problems.

US 2017035949 A1 discloses a device for intrauterine vacuum therapy that includes a pear-shaped fluid-collecting element and a fluid-communicating element. The fluid-collecting element defines an inlet opening at the proximal end and a tubular cavity for receiving a removable guide-rod during transvaginal insertion into a uterine cavity. The fluid-communicating element has a perforated distal end fixed within the fluid-collecting element outside the tubular cavity and has a proximal end adapted for connection to a vacuum-generating system. A method of treating an intrauterine wound or infection is disclosed and includes transvaginally inserting a drain into a uterine cavity and applying a negative pressure to the drain such that the uterus collapses and the inner wall is aspirated against the drain.

US 2016242953 A1 discloses a vaginal laxity/uterine prolapse system. The system includes vaginal laxity/uterine prolapse device and a suction device. The vaginal laxity/uterine prolapse device includes a shaft sized and configured for insertion into a patient's vagina. The shaft includes a plurality of suction ports. The shaft is coupled to a suction device to generate suction through the plurality of suction ports. The suction causes edema and increased blood flow to the vagina walls, resulting in an immediate increase in vaginal tone ("tightness") and over time, with repeated use, causes hypertrophy of the vaginal wall and associated pelvic musculature, resulting in a permanent increase in vaginal tightness, significantly increasing the friction resistance to penile penetration. Use of the vaginal laxity/uterine prolapse system results in immediate and long-term increase in the frictional resistance and improved pelvic and vaginal muscle strength to prevent retrograde migration of the uterus into the vagina (uterine prolapse).

US 2008154183 A1 discloses an irrigation and aspiration system. The system can be configured to aspirate and irrigate alone, sequentially or concurrently. The system can be configured to aspirate and irrigate the nasal cavity. The system can be manually controlled. The system can have removable and easily cleanable reservoirs for aspirant and irrigant.

US 2009281482 A1 discloses irrigation and/or aspiration devices and methods that may be configured to aspirate and irrigate alone, sequentially, or concurrently. The devices and methods may provide a base with a removable head, and adapted for partial or complete separation of the irrigation and aspiration functions. The devices and methods can be configured to aspirate and/or irrigate the nasal and sinus cavities. The devices and methods may be manually and/or automatically controlled. The devices and methods may include removable, and/or replaceable, and/or refillable, and easily cleanable reservoirs for aspirant and irrigant. The device head and/or aspirant reservoir may comprise a diagnostic device, i.e., test device and/or container after use of the devices and methods.

US 4141360 A discloses a vaginal suction apparatus for use particularly during menstrual flow. The device is in the form of a hollow elongated member, having a rounded end portion, defining an inner collection chamber provided proximate the rounded end portion with a screened aperture positionable in the vagina for engagement with the cervix of the uterus. The chamber is closed at its rear end by a wall having a passageway placing the chamber in communication with a suction source, such that by application of suction to the chamber and to the vaginal canal the contents of the uterus are suctioned through the screened aperture into the chamber. Preferably, the rear end wall is removable for emptying the contents of the chamber and for cleaning the chamber. By use of the device a method is provided for regulating and shortening menstrual flow.

US 2016317078 A1 discloses a device and method for measuring skin elasticity that comprises: a probe with one or more holes, a vacuum source, a pressure sensor, and one or more infrared or optical proximity sensors aligned about the one or more holes, wherein the probe further comprises a raised area surrounding the one or more holes; and a processor for recording the deformation of the skin using a control unit comprising a microcontroller connected to the one or more infrared or optical proximity sensors and the one or more pressure sensors, to measure an amount of skin drawn into and out of the one or more holes to determine the distance between the one or more proximity sensors and the skin both inside and outside the probe.

US 2009065565 A1 discloses an application for preventing the reuse of a medical device that includes a disposable medical device and a handle for accepting the disposable medical device. The handle physically supports the disposable medical device. A circuit for identifying the disposable medical device is imbedded within the disposable medical device and a circuit for reading a status of the identification is located in the handle. Whether the disposable medical device has been used is determined based upon the status.

US2010274260A1 discloses a single use, disposable, uterine manipulator device and methods for use thereof. The device comprises a tubular elongate portion defining a longitudinal axis of the device, and having a proximal and a distal extremity, wherein the distal extremity is provided with sealing abutment means for sealingly abutting the cervix, and the distal extremity is further provided with uterine wall suction means distributed around the distal extremity for providing suction to the device, thereby enabling said device to come into suction contact with an inner uterine wall.

### Summary of the Invention

The present invention addresses the need for a treatment of vaginal atrophy that does not involve side effects, can be self-administered, is suitable for use with all women, not just postmenopausal or breast cancer survivor women, and that addresses the underlying cause of the indication without causing irreversible or serious damage to the vaginal epithelium. The solution is based on a device configured to be inserted into the vagina and actuated to cause a mechanical microtrauma to the wall of the vagina. Mechanical microtrauma refers to small deformations to the vaginal wall due to physical interaction between the device and wall that are sufficient to cause inflammation-induced angiogenesis and stimulate proliferation of new endothelial cells and/or generation of collagen matrix. The micro-deformations may include micro tears, punctures, holes, stretches, and cracks in the vaginal wall. Various methods of applying mechanical microtrauma to the wall of the vagina using an insertable device are described herein.

The present invention provides a vaginal atrophy treatment device as defined in claim 1 The device comprises a treatment module configured for insertion into the vagina and having a microtrauma module configured to deliver negative pressure mechanical microtrauma therapy to a wall of the vagina when inserted, wherein the microtrauma module comprises a plurality of apertures in fluidic connection with a vacuum pump, the device being arranged to generate, in use, a negative pressure at the apertures.

The device delivers an array of microtraumas to the wall of the vagina to provide VA treatment. The microtraumas provide a controlled mechanical insult generated using a vacuum to apply negative pressure to the vaginal tissue. The suctioned tissue is strained by the pressure, causing an array of microtraumas that in turn leads to a healing response in the vaginal tissue. The mechanical microtrauma generated in this way produces an inflammatory response that stimulates the production of Hyaluronic Acid (HA).

Known devices include that described in US2018/0296383 A1, which discloses a device that combines ultrasound energy, electrical energy, light, and vacuum to improve the general health function of the penis and the vagina. US2018/0296383 does not disclose that the use of the vacuum therapy can be used to provide effective VA treatment, especially by stimulating angiogenesis as is provided for by the device of the present application. US2018/0296383 A1 discloses only promotion of health function by stimulation of blood flow and sensory nerves. Any use of pressure in US2018/0296383 A1 is merely to physically exercise the muscles of the vagina (paragraph [0051]) and which does not provide microtrauma within the meaning of the present application, and so cannot provide effective VA.

Negative pressure therapy has been suggested previously as a treatment of wounds by Cipolla et al. (Negative pressure wound therapy: Unusual and innovative applications. OPUS, 2008, 12, 15-29) and Saxena et al. (Vacuum-assisted closure: Microdeformations of wounds and cell proliferation. Plastic and Reconstructive Surgery, 2004, 114(5), 1086-1096). The Applicants have discovered that negative pressure therapy is a particularly suitable method of causing mechanical microtrauma to the wall of the vagina. There has previously been nothing to suggest that negative pressure therapy for wound treatment could be used for this purpose. The inventors have found that application of negative pressure to the wall of the vagina stimulates proliferation of new endothelial cells and collagen matrix that will thicken the vaginal wall, reduce atrophy, promote angiogenesis and improve the moisture retaining ability of cells in the vaginal wall, reducing dryness. It is particularly applicable as a therapy for breast cancer patients and postmenopausal women, for whom vaginal atrophy is a recognised side-effect. A key molecule involved in skin and tissue moisture is Hyaluronic Acid (HA) that has a unique capacity in retaining water. HA is a natural polysaccharide, forms an important part of extra-cellular matrix of the vaginal tissue and is present naturally in the vaginal epithelium. It regulates several aspects of tissue repair and regeneration. The synthesis of HA increases in response to inflammation or trauma to the vagina tissue (i.e. the negative pressure will induce a controlled beneficial microtrauma). HA regulates several aspects of tissue repair including activation of inflammatory endothelial cells to enhance angiogenesis to form new blood vessels from existing ones, healing and hydration of damaged tissue. The synthesis of HA is decreased with aging which is associated to reduced oestrogen production. The synthesis of HA reduces after menopause due to reduced levels of oestrogen. The device of the present application will combat this effect by delivering a microtrauma to the wall of the vagina, which will in turn result in an inflammatory response and simulate the production of HA.

The microtrauma module is arranged, in use, to draw tissue of the vaginal wall into the apertures to deliver the microtrauma. This allows microtrauma to be created by straining tissue drawn into each aperture which has been found to provide effective microtrauma for VA therapy. The microtrauma module may be arranged, in use, to stimulate angiogenesis within the tissue of the vaginal wall to provide vaginal atrophy treatment.

The device is arranged to generate a negative pressure of between 200 mmHg and 600 mmHg. Testing performed by the inventors has shown that this provides effective VA treatment without causing excessive tissue damage.

**In** an even more preferred embodiment, the device may be arranged to generate a negative pressure of between 300 mmHg and 450 mmHg at the apertures. This has been found in testing carried out by the inventors to provide an optimal range for effective VA treatment.

The pressure and pressure ranges given herein are to be understood to be the pressure generated when the device is in use, e.g. when VA therapy is being applied by activation of the pump. The pressure generated at the apertures is considered to be the same as that generated by the pump.

The apertures are formed by holes extending from an outer surface of the treatment module. The holes may extend a depth of at least 1 mm (and preferably less than 6 mm) from the outer surface. More preferably the holes may extend a depth of greater than 2 mm (and preferably less than 6 mm). This has been found by the inventors to provide effective treatment. The minimum depth of the holes has been found to be important in avoiding excessive tissue damage by reducing the risk of tissue extending out of the inner end of the hole.

The holes may extend a depth in the range between 3 mm and 4 mm. This has been found to provide a further improved balance of effective microtrauma and risk of tissue damage. This has been found to provide a suitable level of microtrauma to the tissue without causing excessive tissue damage. If the depth is less than this the tissue can extend out of the hole and mushroom on the inside surface of the treatment module causing tissue damage. A depth greater than this range may also result in excessive tissue strain. The holes may extend a depth of 3.5 mm. This has been found to provide optimal therapy.

More preferably the holes may have a maximum cross-sectional size of greater than 1.0 mm and less than 3.5 mm. This has been found by the inventors to provide effective treatment. The inventors have found that the holes must a minimum size in order to draw enough tissue through the apertures to create adequate microtrauma and the desired therapeutic effect.

The holes may have a maximum cross-sectional size in the range between 2 mm and 3 mm. This has been found to provide a further improved balance of microtrauma and risk of tissue damage. The inventors have found that this provides a suitable level of microtrauma without excessive tissue damage. If hole sizes less than the above range are used, less tissue may be drawn into the hole due to the effects of friction which increase as the hole diameter decreases. This may result in an inadequate microtrauma effect required to provide VA therapy. Holes larger than the above range may result in more tissue entering each hole due to lower frictional forces. This results in larger tissue deformations leading to undesired tissue damage. Furthermore, the deformations and associated microtraumas are less concentrated with bigger hole diameters, leading to ineffective VA treatment. The holes may have a maximum cross-sectional size of 2.5 mm. This has been found to provide optimal therapeutic effect.

The portion of the treatment module adapted for insertion into the vagina may have a maximum cross-sectional size in the range between 20 mm and 30 mm. This has been found to provide an advantageous balance between stretching to the vagina during insertion providing additional microtrauma by circumferential stressing, while not making tissue too difficult to provide negative pressure therapy e.g. by making it more difficult for tissue to be deformed and drawn into the apertures.

The treatment module may comprise a support member. The support member may comprise an arrangement of passageways that are fluidly coupled to the vacuum pump. The treatment module may further comprise an outer tip member, the outer tip member comprising a plurality of holes forming the apertures of the treatment module. The outer tip member may be arranged to fit around a distal portion of the support member. The outer tip member and support member may be arranged to form an interlocking engagement (i.e. they have complimentary interlocking shapes) whereby the passageways of the support member are aligned relative to the holes of the outer tip member to form a fluidic connection between them. This aids alignment between the holes and passageways and aids assembly.

The outer tip member may be formed from an elastomeric material. The support member may be formed from a relatively rigid material. A friction fit may be provided between the support member and the outer tip member. This may allow welding or glue to be avoided, and aids manufacture. A seal is also provided between the outer tip and support member to reduce pressure leakage. The elastomeric material may be liquid silicone rubber (LSR) or thermoplastic elastomer (TPE). The support member may be made from a relatively more rigid polymeric material. A stretch fit of the outer tip member over the support member may be provided to aid sealing and coupling between them.

The VA device may further comprise a liquid (e.g. water) trap. The liquid trap may be provided upstream of the vacuum pump. The liquid trap may be arranged to trap liquid discharge drawn through the apertures. The liquid trap may trap water discharge before it gets to the pump. This may help reduce damage to the pump.

The VA device may further comprise a hydrophobic filter. The hydrophobic filter may be arranged to block contaminates drawn though the apertures from reaching the vacuum pump. The hydrophobic filter may comprise a filter module having a hydrophobic filter membrane (e.g. a PTFE membrane). The filter module may be provided in the treatment module, and may be arranged to block contaminates from passing along a flexible lead connecting the treatment module to the pump. The hydrophobic filter may be provided alternatively to the liquid trap.

The treatment module may comprise a proximal handle part and a distal treatment part. The distal treatment part may be adapted for insertion into the vagina during use. The apertures may be provided on the treatment part.

The treatment part may comprise a proximal sealing portion in which none of the apertures are located, and an aperture portion in which the apertures are located. The aperture portion may be located distally to the sealing portion. The sealing portion provides a region that is inserted during use that does not have apertures so that all of the apertures are within the vaginal canal during use. This provides sealing and prevents vacuum leakage during use.

Also disclosed is an example of a device to treat vaginal atrophy comprising a treatment module configured for insertion into the vagina and having a microtrauma module configured to deliver mechanical microtrauma therapy to a wall of the vagina when inserted.

The microtrauma module of the above example is configured to deliver a mechanical microtrauma therapy to the wall of the vagina selected from negative pressure, needle puncture, clamping, heating, cooling, friction or rolling to the wall of the vagina.

The microtrauma module of the above example is configured to deliver negative pressure therapy to the wall of the vagina as discussed above in connection with the first aspect.

The microtrauma module of the above example comprises a plurality of small apertures in fluidic connection with a vacuum pump. The number and size of the apertures employed, and the arrangement of apertures on the microtrauma module, is variable. For example, the apertures may have a maximum dimension of 1 mm to 6 mm. The apertures may be arranged circumferentially around the microtrauma module, or arranged bilaterally in discrete arrays on the microtrauma module. Any number of apertures may be employed, for example at least 10, or 10 -100.

Generally, when the device of the above example is configured to deliver mechanical microtrauma therapy by means of negative pressure therapy, the device will include a vacuum pump operatively connected to the microtrauma module to apply negative pressure to the wall of the vagina through the small apertures. The vacuum pump may be configured to generate a negative pressure of 50 - 800 mmHg during use and may be configured for user adjustment.

In one embodiment, the treatment module comprises a proximal handle part and a distal treatment part (e.g. the handle and treatment parts introduced above). The distal treatment part is dimensioned for insertion into the vagina, and delivery of mechanical microtrauma therapy to the wall of the vagina. The proximal handle part is configured for being held by a user during use. The treatment module is generally an elongated body in which the distal treatment part of the treatment module is optionally cranked with respect to the proximal handle part (for example by about 2 - 45 degrees).

In one embodiment, the distal treatment part is detachable from the proximal handle part. This allows the distal treatment part to be configured for single use, and disposed of after use, while retaining the handle part of multiple uses.

In one embodiment, the device comprises a pressure sensor configured to determine an operational pressure of the negative pressure therapy being applied to the wall of the vagina.

In one embodiment, the device comprises an actuation/controller module. This module may comprise actuation means for the microtrauma module (for example the vacuum pump in the case of negative pressure therapy, or a motor for actuation of microneedles). This module may also include one or more of a graphical user interface, a processor, and a sensor. The actuation/controller module may be located within the microtrauma module (for example the handle part), or it may be located remotely, in a separate base station.

In one embodiment, the actuation/controller module comprises a vacuum pump, a negative pressure sensor, and a processor configured to:
receive data relating to the operational pressure from the negative pressure sensor; compare the detected operational pressure with a reference pressure; and
modify the operational pressure to achieve a target pressure when a sub-optimal operational pressure is detected.

In one embodiment, the device comprises a graphical user interface operatively connected to the actuation/controller module, and configured to graphically display a parameter of the mechanical microtrauma therapy (for example the negative pressure being applied, the length of the treatment period, the time elapsed of the treatment period, the time remaining of the treatment period, etc).

The device comprises a timer configured to switch off the device after a pre-defined treatment period.

In another embodiment, the graphical user interface may comprise a user adjustable timer interface. This may allow the user to set a treatment period. In other embodiments, the treatment period may be pre-programmed. This may reduce user input required, and aid ease of use.

In one embodiment, the treatment module includes an imaging device (for example a video or CCD camera) and optionally an illumination system, configured for imaging the wall of the vagina during use. The device may be configured to display images from the imaging device on the graphical user interface.

In one embodiment, the treatment module includes a moisture sensor configured to sense moisture of the vaginal wall. In one embodiment, the device comprises a processor configured to receive moisture data from the moisture sensor, and process or store the data. The processor may be configured to display data relating the moisture on a graphical user interface forming part of the device, or wirelessly relay the data to a remote computational device.

In one preferred embodiment, the actuation/controller module is a remote actuation/controller module (i.e. provided as a remote base station), operatively connectable to the treatment module and comprising actuating and/or controlling means for the microtrauma module. The actuation/controller module may be operatively connected to the treatment module by, for example, a flexible lead or tubing/conduit. In one embodiment, the treatment module is detachable from the remote actuation/controller module. This configuration allows for use of a disposable treatment module, which can be detached from the remote actuation/controller module after use and disposed of.

The flexible lead may be detachable from the actuation/controller module so that the flexible lead and the treatment module are detachable from the actuation/controller module and are disposable parts. The treatment module may be detachable from the flexible lead so that the treatment module is a disposable part. This allows either the treatment module, or both the treatment module and flexible lead, to be disconnected and disposed of after use and replaced for subsequent uses.

The VA treatment device may further comprise a tamper resistance device. The tamper resistance device may be provided at a connection point between disposable and non-disposable parts of the device (e.g. at a proximal end of the flexible lead where it connects to the actuation/controller module, or at the treatment module where it connects to the flexible cable). The actuator/controller module may be arranged to sense the tamper resistance device and to determine if the disposable parts are being used for the first time based on the tamper resistance device, and prevent activation of the vacuum pump if disposable parts are determined to have been reused. The VA treatment device may be provided with a sensor located in a suitable position to sense or read the tamper resistance device (e.g. at the actuation controller module, or at the distal end of the flexible cable).

The tamper resistance device may comprise a unique identifier, and the device further comprises a sensor arranged to read the unique identifier. The actuation/controller module may be arranged to determine if the flexible lead is being connected for the first time based on the unique identifier. The tamper resistance device may be arranged to be altered or removed during connection of the flexible lead for the first time. It may be a tamper proof cap, seal or foil. The sensor may be arranged to sense the presence or condition of the tamper resistance device, the actuation/controller module being arranged to determine whether the flexible lead is being connected for the first time based on the presence or condition of the tamper resistance device.

In one embodiment, the device (for example the processor) may include communication means for relaying data wirelessly to a remote computational device (for example a mobile phone) via a communication network. The communication network can be the internet, an intranet, or any wired or wireless communication network. For example, the communication network may include a mobile communications network such as a Global System for Mobile Communications (GSM) network, a Code Division Multiple Access (CDMA) network, 3rd Generation Partnership Project (GPP), an Internet Protocol (IP) network, a Wireless Application Protocol (WAP) network, a WiFi network, or an IEEE 802.11 standards network, as well as various communications thereof. Other conventional and/or later developed wired and wireless networks may also be used.

In one embodiment, the device, particularly the treatment module, and more particularly the distal treatment head (e.g. the outer tip member), is formed from a soft lubricious material.

Also disclosed is a method of treatment of vaginal atrophy in a mammal, especially a human, comprising the steps of administering mechanical microtrauma to a wall of the vagina of the mammal, optionally using an insertable device comprising a microtrauma module configured to deliver mechanical microtrauma therapy to a wall of the vagina when inserted. The insertable device may be any device disclosed or claimed herein.

The method comprises administering mechanical microtrauma therapy to the wall of the vagina for a treatment period. The duration of a treatment period may be 1-90, 1-60, 1-30, 5-90, 5-60, or 5-30 minutes. In one embodiment, the mammal is treated once every 1, 2, 3, 4, 5, 6 or 7 days weekly, fortnightly or monthly. The mammal may be suffering from vaginal atrophy. In The mammal may be suffering from vaginal dryness.

The method may comprise the steps of positioning the treatment module at a first position in the vagina, and applying a first dose of mechanical microtrauma therapy, and then re-positioning the treatment module at a second position in the vagina, and applying a second dose of mechanical microtrauma therapy. Repositioning may comprise rotating the device about a longitudinal axis. Re-positioning may comprise adjusting the depth of the device.

Except where mutually exclusive, a feature or parameter described in relation to any one of the above aspects may be applied to any other aspect. As used herein, a range "from value X to value Y" or "between value X and value Y", or the like, denotes an inclusive range; including the bounding values of X and Y.

Other aspects and preferred embodiments of the invention are defined and described in the other claims set out below.

### Brief Description of the Figures

**Figure 1** illustrates a device comprising a treatment module, a remote actuation/controller module, and a lead providing operational communication between the treatment module and remote module.
**Figure 2** is (A) a block diagram illustrating the design of one embodiment of the device of the invention; (B) shows an alternative configuration to that of part (A) where the suction tubing (flexible lead) is reusable and protected by a filter provided in the treatment module.
**Figure 3** illustrates a vacuum therapy treatment module in elevational view (A) and plan view (B).
**Figure 4** illustrates a vacuum therapy treatment module in elevational view: (A) the small apertures are provided as an array disposed on opposite sides of the wall of the module; (B) the small apertures are provided as a single array circumferentially arranged around of the wall of the module; and (C) the apertures are provided in radial bands of holes (the holes may be various shapes other than those shown, for example they may be circular, oval or other shapes).
Figure 5 illustrates the anatomy of the vagina and womb, with a close-up showing inner layer (squamous), connective tissue and muscle layer.
Figure 6 illustrates how devices of the present application may be employed: (A) the treatment module forming part of the device is inserted into the vagina and mechanical microabrasion therapy is delivered by deployment of a plurality of needles into the wall of the vagina; and (B) the treatment module is inserted into the vagina and mechanical microabrasion therapy is delivered by application of negative pressure therapy to the wall of the vagina.
Figures 7A and 7B are illustrations of a device in which the actuation/controller module and power source is located in the handle.
Figure 8 is an illustration of a system comprising a device of the present application and a mobile phone.
**Figure 9** illustrates a device of the present application and a storage case, resting on a bed-side locker.
**Figure 10** illustrates one method of how the flexible lead may connect to the actuator/controller.
**Figure 11** illustrates a device of the present application.
**Figure 12A to 12C** illustrate information that can be graphically illustrated on the LCD screen.
**Figure 13** illustrates an embodiment of the actuator/controller, similar to the embodiment of Figure 12.
**Figure 14** illustrates a treatment module according to another embodiment of the present application in a plan and cross-sectional view.
**Figures 15 to 17** illustrate plan, cross-section and close-up views of embodiments of a treatment module having treatment parts of different shapes and cross-sectional sizes.
**Figure 18A** illustrates plan and cross-section views of an embodiment of a treatment module having apertures with rounded corners.
**Figure 18B** illustrates (A) a close-up view of the embodiment shown in Figure 18a, and (B) the use of rounded edged holes in comparison to sharp edged holes.
**Figure 19** illustrates a VA treatment device according to another embodiment.
**Figure 20** illustrates a treatment module of the device of Figure 19 shown in an exploded view.
**Figures 21** illustrates an outer tip member of the treatment module of Figure 20, the outer tip member being shown in end, top and side views.
**Figures 22** **and** **23** illustrate first and second parts of a support member of the treatment module of Figure 20, the first and second parts each being shown in front, top, back and perspective views.
**Figure 24** illustrates a control/actuation module of the device of Figure 19 in an exploded view.
**Figures 25A, 25B, 25C** show experimental results of the use of a device of the present application.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "vaginal atrophy" refers to the chronic & progressive condition caused by a reduction or absence of oestrogen. It causes the vagina and tissue near it to become dry, thin & inflamed. Vaginal atrophy is prevalent in menopausal women, but more so in postmenopausal Breast Cancer Survivors (BCSs). VA describes a range of symptoms including vaginal dryness, irritation, pain & urinary incontinence & affects a large percentage of menopausal women. This condition disproportionately affects BCSs who experience premature menopause as a result of their cancer treatments. BCSs who receive systemic endocrine therapy (e.g. aromatase inhibitors and Tamoxifen) also experience more severe VA symptoms owing to the oestrogen suppression effect of these therapies. The device and methods of the invention are to treat vaginal atrophy and vaginal dryness.

As used herein, the term "treatment" or "treating" refers to an intervention (e.g. the administration of an agent to a subject) which cures, ameliorates or lessens the symptoms of a disease or removes (or lessens the impact of) its cause(s). In this case, the term is used synonymously with the term "therapy". The treatment may be causal or symptomatic.

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of a negative pressure treatment defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount (pressure and/or length of treatment) will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term subject (which is to be read to include "individual", "animal", "patient" or "mammal" where context permits) defines any subject, particularly a mammalian female subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Treatment module" refers to a part of the device of the invention that is configured for at least partial insertion into the vagina and includes a microtrauma module to deliver mechanical microtrauma therapy to a wall of the vagina when inserted. The module (or at least the part that is inserted into the vagina) will generally have a smooth, atraumatic shape, and a surface comprising the microtrauma module configured to abut the wall of the vagina when inserted. The treatment module may have a distal (transvaginal) part configured for insertion into the vagina ("distal treatment part"), and a proximal part configured for being held by a user during use ("proximal handle part").

"Microtrauma module" means part of the treatment module that is configured to deliver mechanical microtrauma therapy to a wall of the vagina, when the treatment module is inserted into the vagina.

"Mechanical microtrauma" refers to small deformations to the vaginal wall due to physical interaction between the microtrauma module and wall that are sufficient to cause inflammation-induced angiogenesis and stimulate proliferation of new endothelial cells and/or generation of collagen matrix. The micro-deformations may include micro tears, punctures, holes, stretches, and cracks in the vaginal wall. On embodiment of mechanical microtrauma employs negative pressure therapy which is described in more detail below. However, other methods of delivering mechanical microtrauma therapy to the wall of the vagina are envisaged. For example, the microtrauma module may comprise needles (or more preferably microneedles) configured for deployment during use, to create microabrasions in the vaginal wall. The microneedles may be actuated for deployment from a retracted delivery position within the device to a deployed position during use where the microneedles penetrate the wall of the vagina. In another embodiment, the microtrauma module may comprise means for stretching the wall of the vagina to create micro tears or fissures in the wall, for example means for gripping a section of the wall at each end of the section, and then stretching the wall. In another embodiment, the use of heating or cooling may be employed to generate mechanical microtrauma in the wall of the vagina. In another embodiment, the mechanical microtrauma module may comprise means for applying friction or mechanical deformation (rolling) to the wall of the vagina.

"Negative pressure therapy" refers to the process of forming micro-deformations in the wall of the vagina by the application of a vacuum to the wall of the vagina, where the micro-deformations are sufficient to stimulate proliferation of new endothelial cells and collagen matrix that will thicken the vaginal wall, reduce atrophy and improve the moisture retaining ability of cells in the vaginal wall, reducing dryness. One embodiment of the device of the invention applies negative pressure therapy to an area of the wall of the vagina by means of a plurality of small apertures, typically an array of small, closely packed apertures, that are fluidically connected to a vacuum pump in the device. The application of negative pressure therapy to wound healing is described in the literature, for example Cipolla et al. (Negative pressure wound therapy: Unusual and innovative applications. OPUS, 2008, 12, 15-29) and Saxena et al. (Vacuum-assisted closure: Microdeformations of wounds and cell proliferation. Plastic and Reconstructive Surgery, 2004, 114(5), 1086-1096). Negative pressure wound therapy facilitates healing by improving the rate of angiogenesis, endothelial proliferation, capillary blood flow and decreasing interstitial edema (amongst other things). This approach is applicable to the vaginal wall where it causes a similar beneficial microtrauma effect. The negative pressure produces a controlled, beneficial microtrauma to the vaginal wall to induce a beneficial inflammation cascade of angiogenesis and neo-collagenesis (collagen renewal) which is a recognised molecular pathway. Negative pressure therapy is supported by literature to induce angiogenesis & endothelial proliferation that are the mechanisms of action required to rejuvenate the vaginal wall to address vaginal atrophy. Vaginal lubrication originates from the lamina propria, which contains elastic fibres, blood vessels, lymphatic & nerves, as well as glands that secrete mucus and serous fluids. The lamina propria of the atrophic vagina has decreased extracellular matrix components, reduced vascularization & water-retaining capacity. The device and methods of the invention induce angiogenesis of the lamina propria to create micro-vascularization & new vessel formation. This in turn will produce moisture & lubrication, simultaneously collagen remodelling of the connective tissue & elastin fibres of the vaginal wall & restoring the vaginal mucosa & rehydrating the vaginal walls. The synthesis of HA increases in response to inflammation or trauma to the vagina tissue (i.e. the tip with the perforations will induce a controlled beneficial microtrauma). HA regulates several aspects of tissue repair including activation of inflammatory endothelial cells to enhance angiogenesis to form new blood vessels from existing ones. The synthesis of HA is decreased with aging which is associated with reduced oestrogen production.

"Small apertures" should be understood to mean holes that are sufficiently small to deliver effective negative pressure to the wall of the vagina. Typically, the holes have a maximum diameter of about 1-3 mm, 2-3 mm, or preferably about 2.5 mm. Typically the holes are circular or oval, although other shapes of holes may be employed, for example square or rectangular, provided that they are sufficiently small.

"Remote actuation/controller module" refers to an actuation/controller module that is separate from the treatment module and generally connected by a connecting lead. It is also referred to herein as a remote "base station". The remote actuation/controller module generally includes means for actuating the mechanical microtrauma module, for example a pump, motor, heater, a graphical user interface, and a processor for receiving data relating the operation of the treatment module or the treatment.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

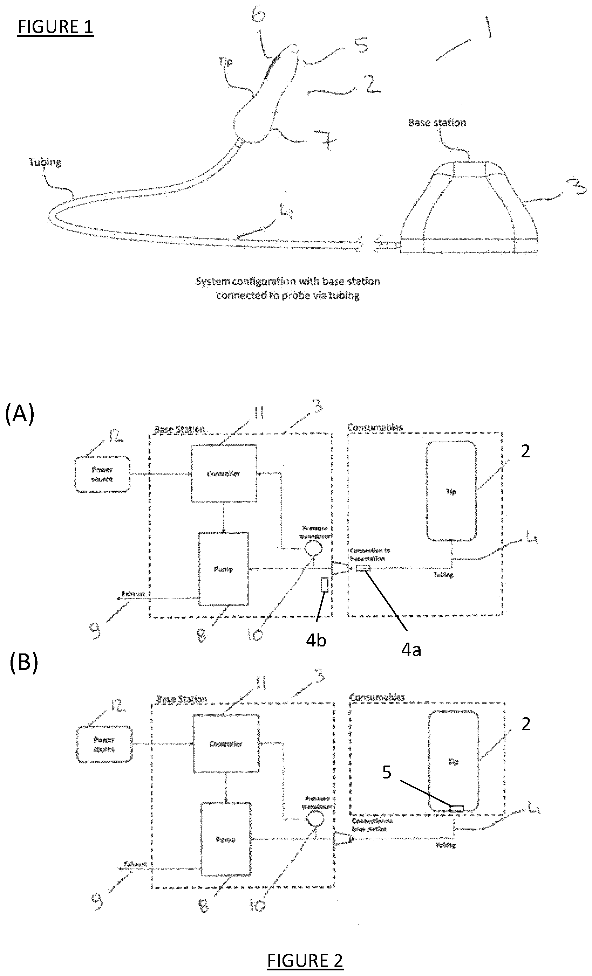

Referring to the drawings, and initially to Figures 1 to 3, there is illustrated one embodiment of a device of the invention, indicated generally by the reference numeral 1. In this embodiment, the device is configured for delivery of mechanical microabrasion therapy by means of negative pressure therapy. The device 1 comprises a therapeutic (or treatment) module 2, a remote actuator/controller module 3, and a flexible lead 4 providing operational communication between the therapeutic module 2 and remote module 3. The therapeutic module 2 comprises a distal treatment part 5 having a curved atraumatic shape, and a microtrauma module provided in the form of two arrays of small apertures 6 disposed on opposite sides of a sidewall of the distal part, and a proximal part 7 configured to be held by the user during use of the device. The actuation/controller module 3 comprises a vacuum pump 8 fluidically connected via the lead 4 to the array of small apertures 6, and to an exhaust 9, a pressure transducer 10 configured to detect in real time the vacuum being drawn by the pump 8, and a processor 11 configured to receive pressure data from the pressure transducer 10. A power source 12 is operatively connected to the processor, pump and pressure transducer, which power source may be mains electricity or a battery (not shown). The processor is configured to receive pressure data from the transducer 10, and process the data. The processor may be pre-programmed to apply a level of negative pressure therapy, and may be configured to adjust the pump to modulate the pressure according to pressure readings received from the pressure transducer. The processor may be operatively connected to a graphical user interface (GUI) to display pressure readings on the GUI.

In any embodiment described herein, both the treatment module 2, or both the treatment module 2 and the flexible lead 4 may be detachable from the actuator controller module 3. One or both of the treatment module 2 and the flexible lead 4 may then be disposable once detached.

In the embodiment shown in Figure 2 part (A), the flexible lead 4 is detachable from the actuation/controller module 3. The flexible lead 4 and treatment module 2 are therefore disposable, and can be replaced for further uses (i.e. they are consumables). In the embodiment shown in Figure 2 part (B), the flexible lead 4 is detachable from the treatment module 2 so that only the treatment module is disposable. In this embodiment, the treatment module 2 comprises a filter 5 (e.g. a hydrophobic filter module as will be described later) that is arranged to block contaminates drawn though the flexible lead 4 from reaching the vacuum pump 8.

In any embodiment described herein, the VA treatment device may further comprise a tamper resistance device arranged to prevent repeated use of any components that are disposable. In the embodiment of Figure 2 part (A), the proximal end of the flexible lead 4 (i.e. that connected to the actuation/controller module 3) has a tamper resistance device 4a. The actuator/controller module comprises a sensor 4b arranged to sense the tamper resistance device to determine whether the flexible lead has been used before, or if it is being used for the first time. The actuation/controller module 3 is arranged to prevent activation of the treatment if the flexible lead 4 is determined to have been connected before.

In one embodiment, the tamper resistant device 4a comprises a unique identifier that can be read by a sensor 4b provided at the actuation/controller module 3. The actuation/controller module 3 is arranged to determine if the flexible lead has been used before based on the unique identifier. For example, the actuation/controller module 3 may record the unique identifier in a memory when the flexible lead 4 is connected. The actuation/controller module 3 may then compare subsequently read unique identifiers to those read during previous uses in order to determine if the flexible lead/ treatment module are being reused or are being connected for the first time. The unique identifier may take the form of a printed ID code (e.g. barcode, QR-code) or may be stored on a chip or magnetic strip or the like.

In other embodiments, the tamper resistance device 4a is configured to be altered or removed during the process of the connection of the flexible lead 4. For example, the tamper resistance device 4a may comprise a tamper proof cap, seal or foil that is broken/removed when the flexible lead 4 is connected for the first time. The actuation/controller module 3 is arranged to sense the presence or condition of the tamper resistance device 4a to determine whether the flexible lead 4 is being reused via a suitable sensor provided at the connection point.

While the tamper resistance device 4a is shown at the proximal end of the flexible cable 4, it may be provided at other locations within the device. For example, the tamper resistance device 4a may be provided in the treatment module 3, with a corresponding sensor provided in a connector at the end of the flexible lead 4. This arrangement is suitable for where only the treatment module is disposable.

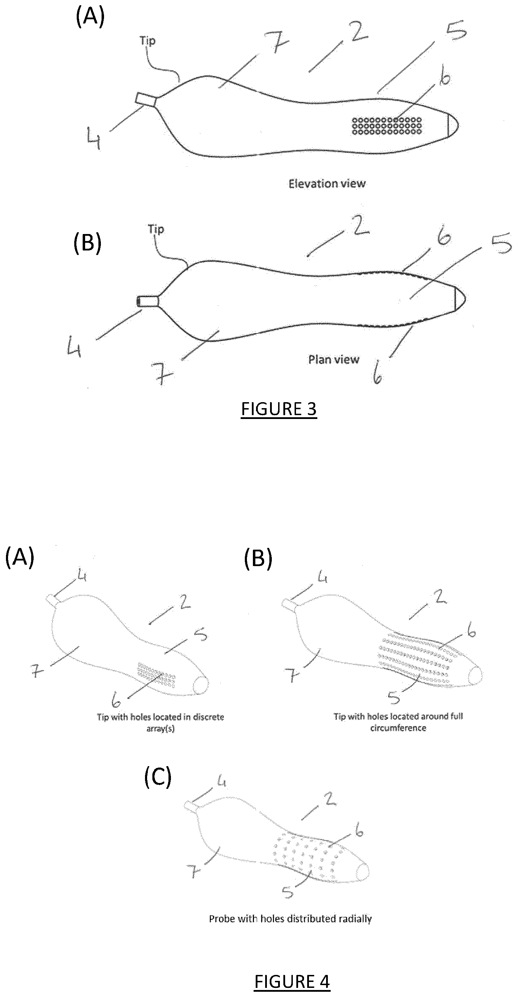

Figure 4 illustrates three embodiments of the treatment module in which the small apertures are provided as an array disposed on opposite sides of the wall of the module (A), and the small apertures are provided as a single array circumferentially arranged around of the wall of the module (B). (C) shows an embodiment in which holes are distributed radially. It will be appreciated that the small apertures may be provided in other arrangements, including multiple arrays of apertures.

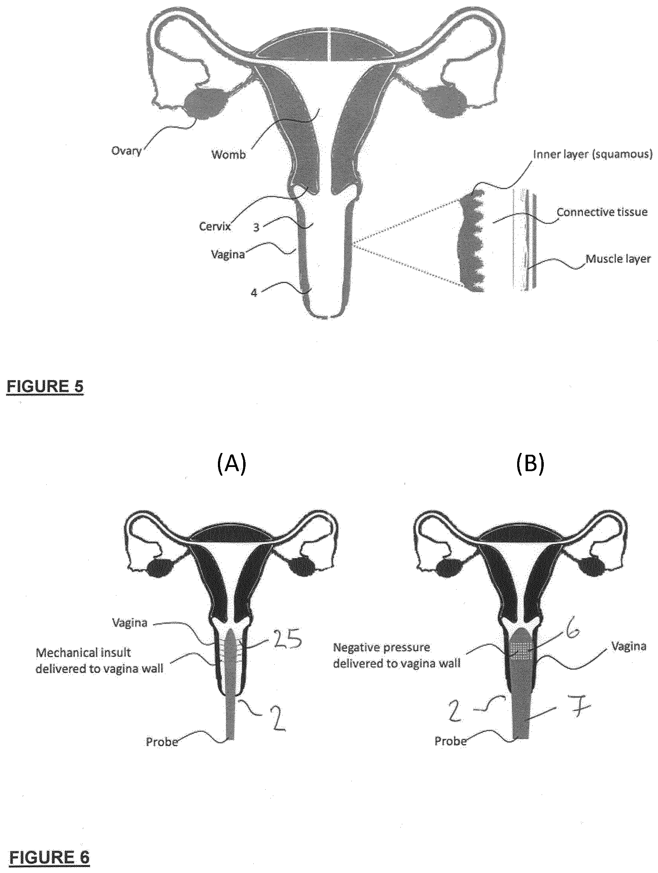

The use of the device of the invention is illustrated in Figures 5 and 6, where Figure 5 illustrates the anatomy of the womb and vagina of a female human, and Figure 6 illustrates the distal treatment part of the treatment module of the device inserted into the vagina and actuated to administer mechanical microtrauma therapy. In Figure 6B (image on right), the device comprises a circumferential array of small apertures configured to deliver negative pressure therapy to the wall of the vagina in contact with the apertures. In Figure 6A (image on left), the device comprises an array of deployable needles 25 configured for deployment into the wall of the vagina. In both cases, the treatment results in the creation of micro-deformations in the wall of the vagina that are sufficient to cause angiogenesis and stimulate proliferation of new endothelial cells and/or generation of collagen matrix, and thereby treating vaginal atrophy.

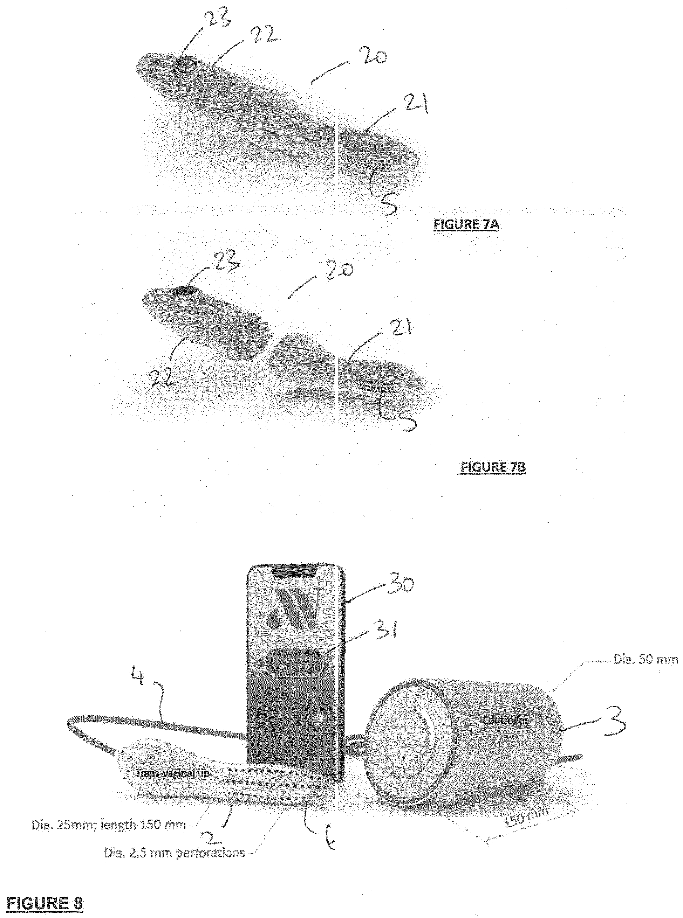

Referring to Figures 7A and 7B, an alternative embodiment of the device of the invention is illustrated, in which parts identified with reference to the previous embodiments are assigned the same reference numerals. In this embodiment, indicated by the reference numeral 20, the device is provided as a single elongated body having a distal treatment part 21 with arrays of apertures 5 and a proximal handle part 22, which parts are configured for detachment. The handle part 22 includes a vacuum pump, processor, pressure sensor, a battery (not shown), and an on/off button 23. The distal treatment part is a single use, disposable part, which can be discarded after use. This embodiment of the device may be provided in a modular format, comprising a single handle part, and a number of disposable distal treatment parts.

Referring to Figure 8, there is illustrated a device of the invention in which parts identified with reference to the previous embodiments are assigned the same reference numerals. The device comprises a treatment module 2 and a remote actuator controller 3 connected by a lead 4, as described previously. The actuator/controller is configured to relay data to a mobile phone 30, and the phone comprises downloadable software (i.e. an "app") configured to receive and process data received from the actuator/controller, and display information relating to the operation of the device and the therapy on the phone screen 31. In the illustration, the phone provides information in relation to the status of the therapy (i.e. treatment in progress) and in relation to the treatment time remaining.

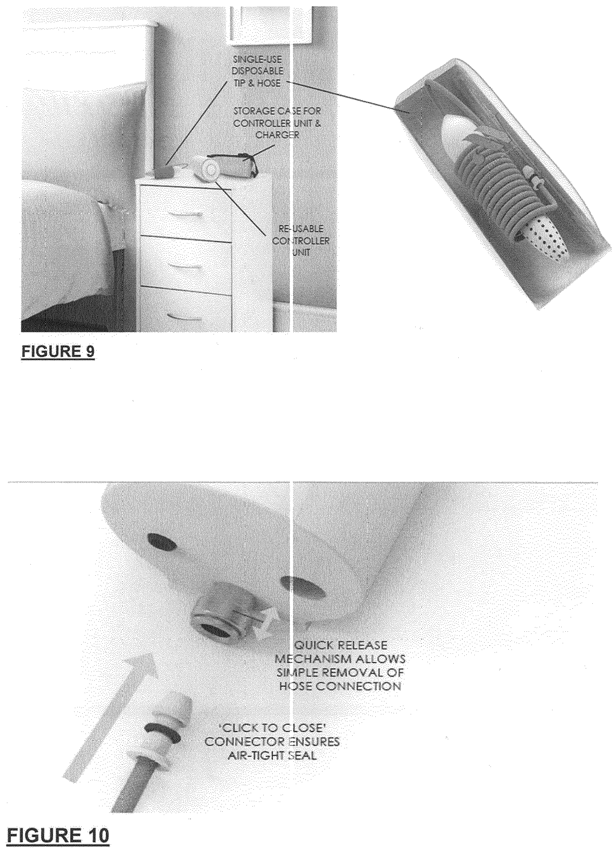

Referring to Figure 9, there is illustrated a device of the invention and a storage case, resting on a bed-side locker. The device comprises a remote re-usable actuator/controller, a disposable treatment module and flexible lead. A storage case of the actuator controller is provided, along with a storage case for the treatment module and flexible lead.

Referring to Figure 10, there is illustrated one method of how the flexible lead may connect to the actuator/controller, having a friction-fit "click-to-close" connecting mechanism, and a quick release mechanism to allow for easy and fast disconnection of the lead and the actuation controller.

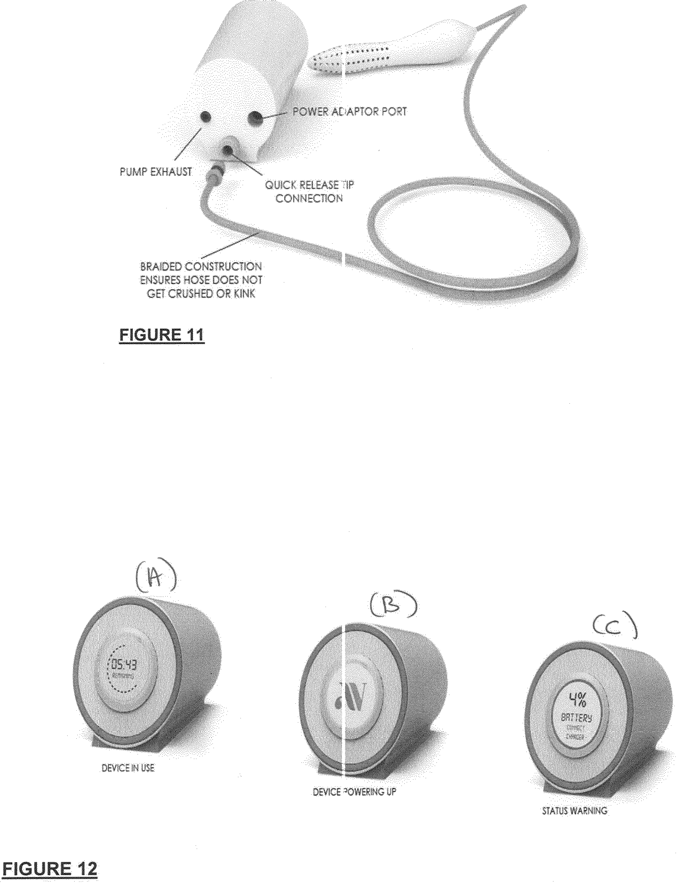

Referring to Figure 11, there is illustrated a device of the invention, showing an end face of the actuator/controller having a port for receipt of the flexible lead, a pump exhaust vent, and a power adaptor port. Also illustrated is a flexible lead comprising a braided sheath which helps avoid crushing or kinking of the lead.

Referring to Figures 12A to 12C, there is illustrated information that can be graphically illustrated on the LCD screen including information relating to a treatment period (A), information relating to the operational status of the device (B), and information relating to the remaining battery power of the device (C).

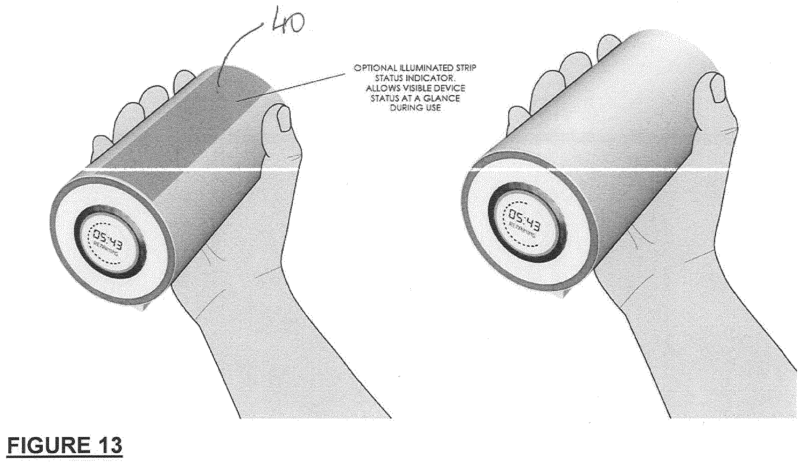

Referring to Figure 13, there is illustrated an embodiment of the actuator/controller, similar to the embodiment of Figure 12, in which the actuator/controller comprises a graphical display disposed on a side of the actuator (in this case a strip of illumination 40) that can indicate the device status at a glance.

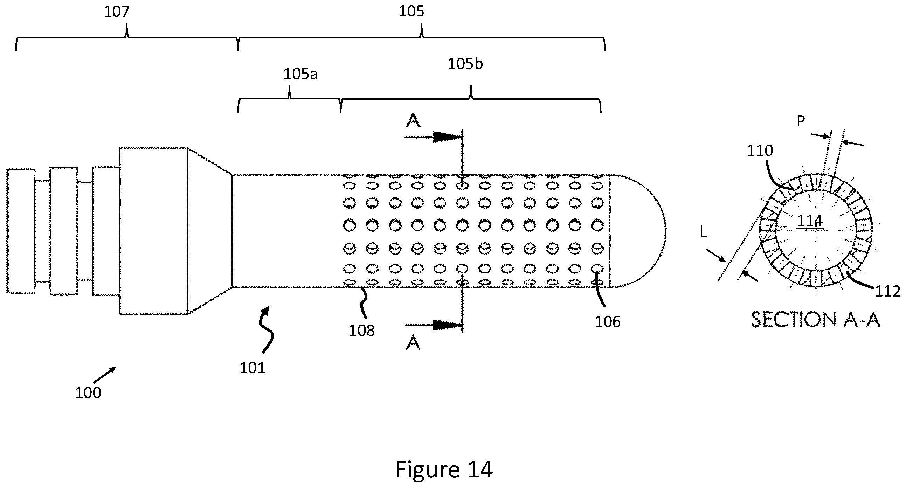

Another embodiment of a VA treatment device 100 is illustrated in Figure 14. In this embodiment, the treatment device 100 is configured to deliver mechanical microtrauma therapy in the form of negative pressure therapy to the wall of the vagina.

As can be seen in Figure 14, the device 100 comprises a treatment module 101 configured for insertion into the vagina. The treatment module 101 has a distal treatment part 105 that is insertable into the vagina and a proximal handle part 107 by which it may be manipulated by the user similarly to other embodiments described herein. Corresponding reference numerals have been used for parts common to other embodiments to aid explanation. Any of the features described in connection with other embodiments may apply to the embodiment of Figure 14.

The treatment module 101 comprises a plurality of apertures 106 in an array over at least part of the outer surface 108 of the treatment part 105. The apertures 106 shown in Figure 14 are disposed evenly over the entire circumference of the treatment part 105. In other embodiments, the apertures 106 may be disposed in discrete arrays, may have a non-uniform distribution or may be arrayed around only part of the circumference.

The distal treatment part 105 of the treatment module 101 comprises a sealing portion 105a (illustrated in Figure 14) that is an axial part of the portion of the device that is inserted into the vagina during use, but which does not include any of the array of apertures 106. The apertures are located only in an aperture portion 105b located distally to the sealing portion. The sealing portion is located at the distal end of the treatment part as illustrated. By forming a region with no apertures the sealing region helps ensure that all of the apertures are in contact with the vagina wall when the treatment part is inserted, and that no vacuum pressure leaking occurs due to apertures not being in the vaginal canal during use when the vacuum pressure is applied.

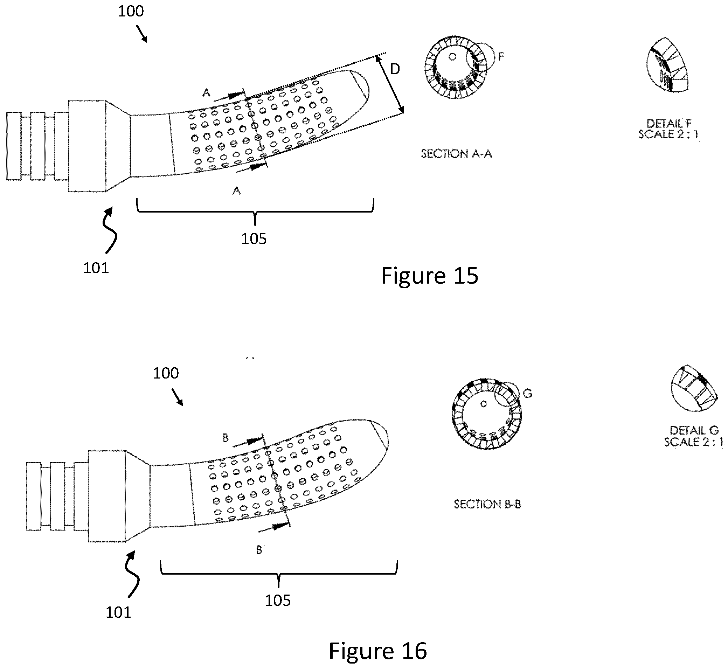

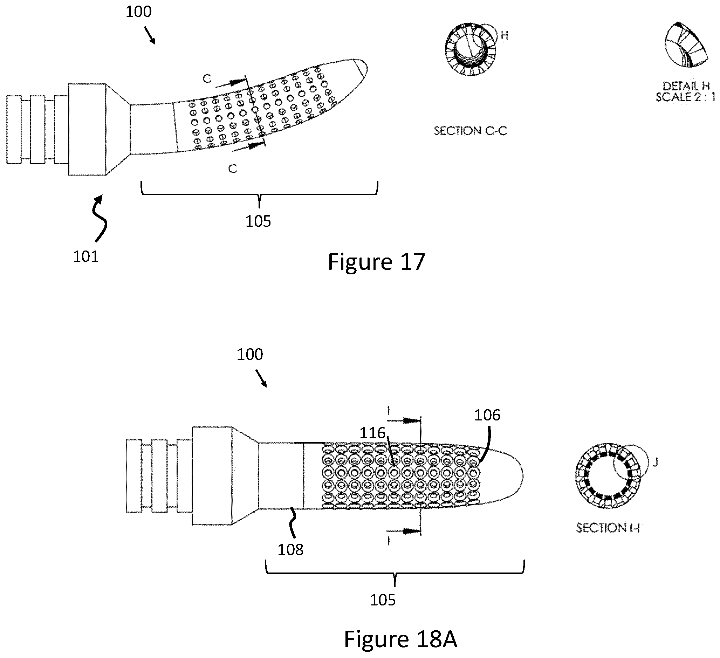

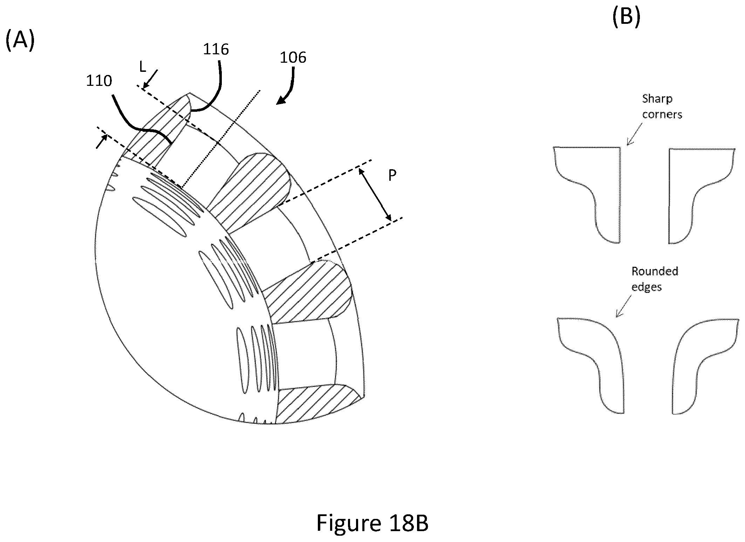

The treatment module 101 comprises an elongate hollow member having an atraumatic shape (that may be straight as shown in Figures 14 and 18; or curved as illustrated in Figures 15 to 17). The elongate hollow member forms the treatment portion 105. As can be seen in the cross section shown in Figure 14, the apertures 106 are formed by through holes 110 extending through a wall 112 of the treatment module 101 that open into an internal cavity 114 within the hollow member. The cavity 114 is in fluidic communication with a vacuum pump (not shown in Figure 14) to generate a negative pressure within the cavity 114 and at the apertures 106. In this embodiment, the device 100 further comprises an actuation/controller module (not shown in Figure 14) as described with respect to other embodiments. The vacuum pump is located within the actuation module and is fluidly connected to the treatment module via a flexible lead as described in connection with Figure 1. The flexible lead takes the form of a flexible length of tubing connecting treatment module and the actuation/controller module. The vacuum pump can however be located within the treatment module 101 (e.g. within the handle part 107) as described in connection with Figures 7A and 7B.

The VA treatment device 100 of the embodiment shown in Figure 14 is configured to deliver mechanical microtrauma therapy by drawing tissue into and through the apertures 106. When in use, negative pressure provided at the apertures 106 causes adjacent tissue close to or in contact with the outer surface 108 of the treatment module 101 to be drawn through the apertures 106 so that the tissue elongates within the holes 110. The mechanical microtrauma is thus created by the straining of the tissue that is drawn into each aperture 106.

In order to draw tissue into the apertures a negative pressure (i.e. a pressure less than the ambient atmospheric pressure) is generated at the apertures by the vacuum pump. In various embodiments the pressure generated by the pump at the apertures is at least about 50 mmHg, and preferably less than 800 mmHg. In yet other embodiments, it is greater than about 100 mmHg, and preferably less than 800 mmHg. In some embodiments the pressure may be in the range between 50 mmHg and 600 mmHg. The pressure ranges in this paragraph are generally suitable for providing negative pressure at the vaginal tissue, but without causing tissue damage. Other pressure ranges defined elsewhere herein may be used.

In the presently described preferred embodiment, a pressure of between about 200 mmHg and 600 mmHg is generated during use. This pressure has been found by the inventors to provide an advantageous level of tissue microtrauma when applied to the vagina wall which in turn provides the desired VA therapy.

The inventors have found that the operating pressure has a significant effect on the microtrauma created by the device 100. At low pressures the deformation of the vagina tissue is limited and therefore minimal strain is generated. This results in a small microtrauma being developed. This may not be significant enough to generate the desired healing response and associated rejuvenation of the vagina tissue. At high vacuum pressures the tissue is drawn deep into each aperture. This may produce excessive elongation of the tissue causing undesired tearing and or bleeding. Moreover, if the tissue is excessively elongated within the holes it may bulge out on the inner surface of the treatment part, creating a mushroom-like effect. This can again result in tearing of the vaginal tissue and/or bleeding. Tearing and bleeding can lead to negative clinical outcomes including scarring or ulcer formation.

In other embodiments, the pressure may be in the range between 300 and 450 mmHg. This has been found to provide a yet more optimal therapeutic effect.

In the presently described embodiment, the holes have a cross-sectional size (e.g. maximum diameter) labelled P in Figures 14 and 18. The inventors have found that the behaviour of tissue being drawn into each hole is further affected by the hole inlet size, in addition to the pressure and other factors. The cross-sectional size of the apertures therefore has a significant effect on the microtrauma and corresponding clinical efficacy and safety. In various embodiments, the holes have a maximum cross-sectional size of at least 0.5 mm, and preferably less than 4 mm. In other embodiments, the holes have a maximum cross-sectional size of greater than1.0 mm and preferably less than 4 mm.

In the described preferred embodiment, the hole diameter is in the range of between 2 mm and 3 mm. This has been found to provide more optimal microtrauma. In yet other embodiments, an optimal diameter of about 2.5 mm may be used. This has been found by the inventors to provide optimal treatment. The inventors have found that a range of between about 2 mm to 3 mm provides an optimal hole diameter which maximises the level of microtrauma without risking complications, for example tearing of the tissue and associated bleeding. If hole sizes less than the above range are used, the result may be less tissue being drawn into the hole due to the effects of friction which increase as the hole diameter decreases. This may result in an inadequate microtrauma effect required to provide VA therapy. Holes larger than the above range may result in more tissue entering each hole due to lower frictional forces. This results in larger tissue deformations leading to undesired tissue damage. Furthermore, the deformations and associated microtraumas are less concentrated with bigger hole diameters leading to ineffective VA treatment. The range of hole cross-sectional sizes has been found by the inventors to provide advantageous results, particularly in combination with the pressure ranges defined herein.

In the embodiment shown in Figure 14 the holes are generally cylindrical in shape and have a constant diameter along their length. In other embodiments, the holes may have other shapes, they may for example be oval in cross-sectional shape. In other embodiments the diameter of the holes may vary along their length. In such an embodiment, the diameter of the holes is measured in the plane of the aperture (i.e. not including any larger or rounded part of the aperture at the external wall of the treatment part as shown in Figure 18). The cross-sectional sizes specified for the apertures given herein are measured for the hole or channels forming the apertures as shown in the inset of Figure 18 at the plane of the aperture.

Referring to the cross-section shown in the insert of Figure 14, the holes 110 which form the apertures 106 have a length or depth labelled L, by which they extend from the outer surface 108 of the treatment model through the wall 112. The length L is measured along the axial centreline of the holes from a point level with the outer surface 108 of the wall 112 of the treatment part 105 to a point level with the inner surface of the wall 112 at which the holes 110 open to the internal cavity 114. In various embodiments the holes extend a depth of at least about 1 mm (and preferably less than 6mm). In other embodiments, the holes extend a depth of greater than about 2 mm (and preferably less than 6 mm). The inventors have found that the depth of the holes also has an effect on the microtrauma created. The inventors have found that a hole depth less than the above range risks the tissue quickly reaching the inside surface of the hollow member where it can be pulled out by the vacuum, forming a mushroom effect on the inside surface. This can lead to too much deformation of the vaginal tissue and associated complications.

In the presently described preferred embodiment the hole depth is between 3 mm and 4 mm. The inventors have found this to provide a more optimised level of microtrauma suitable for VA treatment. In yet other embodiments, a hole depth of 3.5 mm is provided. This has been found by the inventors to provide optimal treatment. The inventors have found that a hole depth greater than the above range may cause excessive damage, i.e. it may tear the tissue. The range of hole depth defined in this paragraph and the paragraph above have been found by the inventors to provide advantageous results, particularly in combination with the pressure ranges defined herein.

In other embodiments, different hole geometries may be provided. The depth of the holes is defined as the length of a channel created by the holes in which contained tissue elongation may occur. In other words, the hole depth is the distance that tissue may extend from the outer surface 110 before being no longer contained by the internal walls of the hole (e.g. by opening into the cavity) or by coming into contact with an end wall of the hole.

Figures 15 to 17 illustrate embodiments of the device 100 having various different shapes of the treatment part 105. In these embodiments, the treatment part 105 has a curved shape rather than the straight shape shown in Figure 14.

In Figures 15 to 17, various cross-sectional sizes of the treatment part 105 are illustrated. The maximum cross-sectional size D of the treatment part may be in the range between 20 mm and 30 mm. The inventors have found that a large diameter (for example greater than 30 mm) will cause stretching of the vagina wall circumferentially. This will in turn make it more difficult to deform the tissue and draw it into each aperture so that the negative pressure therapy can be suitably provided. Larger diameters however mean that the tissue will be already stressed circumferentially by the treatment module acting as a dilator and therefore, some level of trauma will already be delivered to the tissue. Correspondingly, a small diameter (e.g. less than 20 mm in diameter) will not circumferentially stretch the vaginal canal sufficiently and tissue will be easily drawn into each aperture because there is no resistance from the tensioning of the tissue. If the tissue is not in tension it is more easily drawn into the apertures because there is nothing to resist it. If the diameter is excessively narrow, the negative pressure may not be adequately applied to the tissue surface, and it may be unable to draw tissue into the apertures at all. The inventors have found that a cross sectional size in a range of 20 mm to 30 mm provides an advantageous balance of these factors (e.g. for a human subject). Other sizes may however be contemplated for other applications.

In the embodiments illustrated in Figures 14 to 17 the treatment part is circular in cross-section. In other embodiments, the cross-section may be non-circular. In some embodiments, the cross-sectional size (or shape) may vary along the length of the treatment part 105, as illustrated in Figure 16. The cross-sectional size of the treatment part 105 is taken to be the maximum cross-sectional size at any point along the length of the insertable tip section (e.g. the length of the treatment part that is configured for insertion during use).

Another embodiment of the VA treatment device is illustrated in Figures 18a and 18b. In this embodiment, the holes 110 forming the apertures have rounded edges 116 where they meet the outer surface of the treatment part. In the embodiment illustrated, all of the inlets to the holes 106 have rounded edges. In some embodiments however, only some of the holes 106 may have rounded edges at positions where there is greater risk of damage being caused to tissue when drawn through the apertures. The inventors have found that the inlet geometry of each of the holes at the outer surface 108 of the treatment part 105 will also have an effect on how the tissue is drawn into each hole. Rounding of the inlet to each hole 106 reduces the mechanical loading on the tissue as it is drawn into the hole 106 and elongates as it is pulled down the channel of the hole. The rounded edges help to reduce friction and strain in the vagina tissue as it is drawn past the hole edge. This reduces the risk of tearing of the tissue. The cross-sectional sizes specified for the apertures given herein are measured for the holes or channels forming the apertures as shown in the inset of Figure 18 at the plane of the aperture without including the rounded edges of the holes.

Although the embodiments shown in Figures 14 to 17 have holes without rounded edges it is to be understood that this is an example only, and in preferred embodiments rounded corners are provided. The inventors have found it is desirable to eliminate sharp corners on the holes which would be a potential source of trauma should the device or patient move during treatment. The use of rounded edges to the holes is illustrated in Figure 18b (B), with an illustration of sharp edged holes for comparison. As can be seen in the figures, the rounded corners are advantageous to avoid tissue damage.

In various embodiments, the outer surface of the treatment part is formed from a material having any one or more of: a Shore hardness of 40A and a frictional coefficient of sliding less than or equal to 0.4. Other material properties may be provided.

The inventors have found that the ranges of pressure, hole depth, aperture diameter and treatment module cross-section described above provide an advantageous therapeutic effect. Each of these factors may however be used independently to provide an advantageous effect and are not all essential to provide the desired mechanical microtrauma.

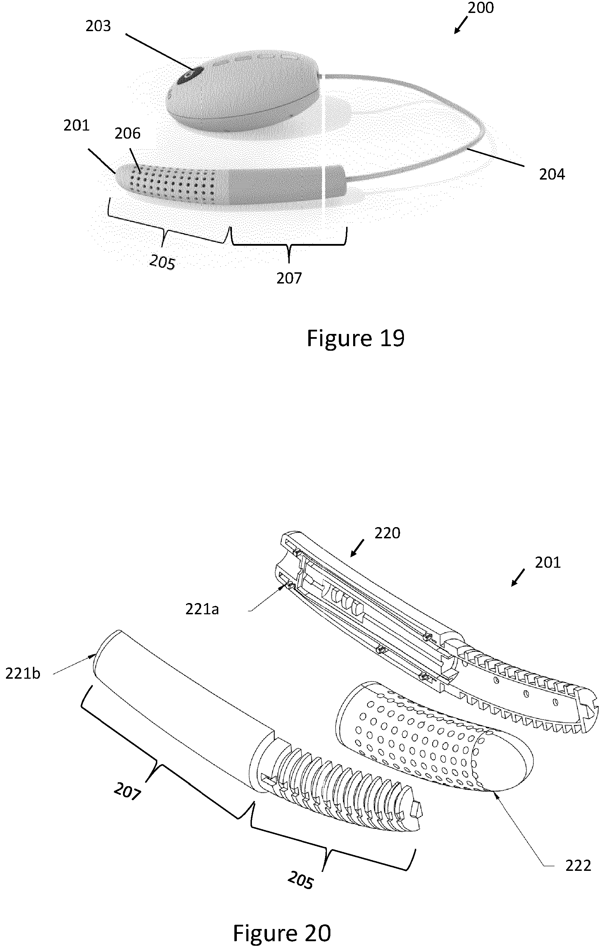

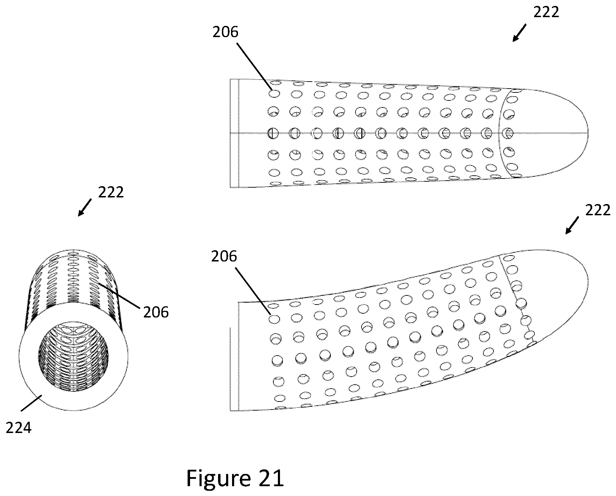

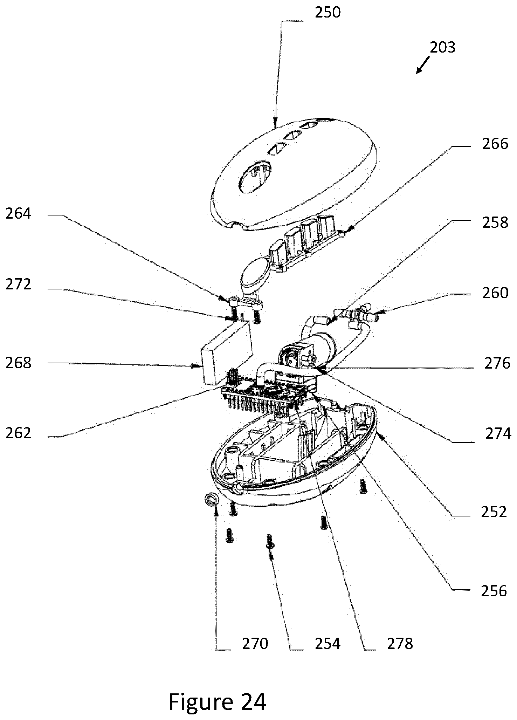

Figures 19 to 24 show another embodiment of a VA treatment device 200. The treatment device 200 comprises a treatment module 201, an actuation/controller module 203 that is coupled to the treatment module 201 by a flexible lead 204 as described in connection with other embodiments herein. The treatment module is shown in more detail in Figures 20 to 23, with the actuation/controller module 203 shown in Figure 24. The treatment module 201 has a distal treatment part 205 that is insertable into the vagina and a proximal handle part 207 by which it may be manipulated by the user similar to other embodiments described herein. The treatment part comprises a plurality of apertures 206, similar to other embodiments described herein that are fluidly connected to a vacuum sourced (e.g. a pump) provided within the actuation/controller module 203. Anything described in connection with other embodiments elsewhere herein may also apply to the embodiment of Figures 19 to 24 (for example, the features described above in connection with the embodiments of Figures 14 to 18 may apply to the embodiment of Figures 19 to 24, such as the pressure ranges and aperture arrangement, sizes, hole depths and geometry etc.). Referring to Figure 20, the treatment module 201 comprises a support member 220 (e.g. a support frame/scaffold) and an outer tip member 222 (e.g. an outer "sock/sleeve"). In the present embodiment, the support member 220 is formed from two parts, a first part 221a and a second part 221b. A distal portion of the support member 220 forms an inner part of the distal treatment part along with the outer tip member, and a proximal portion of the support member 220 forms the handle portion. The outer tip member 222 has an atraumatic elongated shape for insertion into the vagina, and may be shaped as described in relation to any other embodiment herein. The outer tip member 222 has a cavity extending along its axial length (as can be seen in Figure 21) which is open at its proximal end. The outer tip member 222 is arranged to fit over the distal portion of the support member such that the support member is received within the cavity.

A friction fit is provided between the outer tip member 222 and the support member 220. This may aid manufacture as glue or welding is not required to join the components. The outer tip member 222 is formed from an elastomeric material such as a Liquid Silicone Rubber (LSR) or Thermoplastic Elastomer (TPE). Other suitable elastomeric materials may be used. The use of an elastomeric material may improve the friction fit between the cover member 222 and the support member 220, as a tight fit may be provided. In some embodiments, a stretch fit of the outer tip member 222 over the support member 220 may be provided to aid sealing and coupling between them. A seal between them may also be provided by the use of the elastomeric material to reduce pressure loss or liquid ingress. The distal portion of the support member 220 has a shape corresponding to that of the outer tip member 222 so that it will keep its shape during use. The support member 220 is made from a relatively more rigid material compared to the outer tip member to provide structure strength of the treatment part to allow insertion.

The outer tip member 222 is illustrated separately in Figure 21. The outer tip member comprises the plurality of apertures 206 via which microtrauma therapy is delivered. The apertures are formed by through holes which extend through the wall 224 of the outer tip member. The holes may have any of the sizes, shapes and depths as described elsewhere herein.

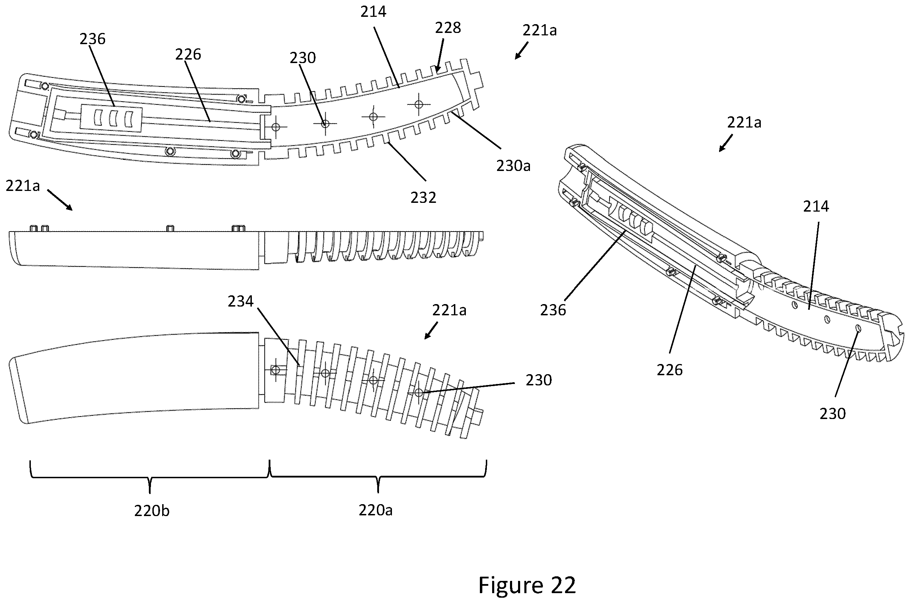

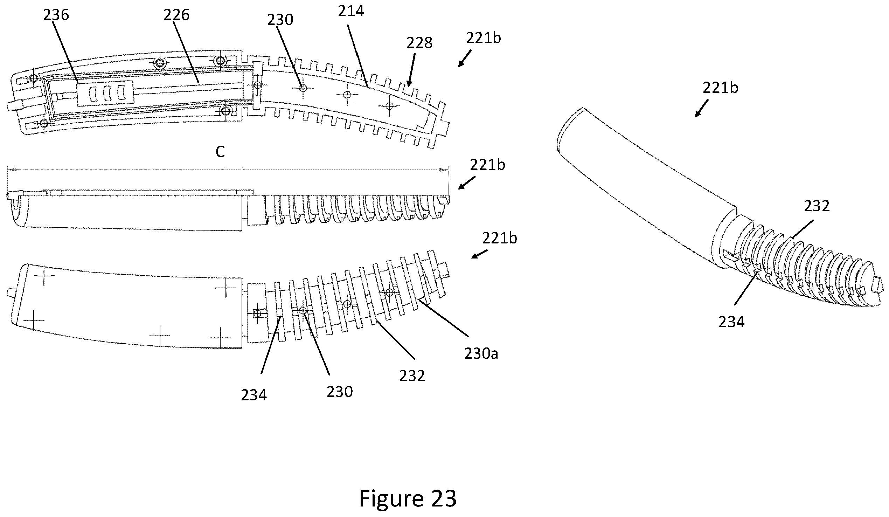

The support member 220 is illustrated separately in Figures 22 and 23, which show the first part 221a and the second part 221b of the support member in various views, respectively. The support member 220 comprises a distal portion 220a adapted for insertion into the vaginal canal during use and so forms part of the treatment portion of the treatment module. The support member 220 further comprises a proximal portion 220b that forms the handle portion of the treatment module. The outer tip member 222 fits over the distal portion 220a as shown in Figure 20. In the present embodiment, the support member 220 has a length C of about 160 mm. Other sizes may be contemplated with this given as an example only.

The support member comprises an internal cavity 214. The cavity 214 is defined by a wall forming the distal portion 220a of the support member 220. The cavity 214 is in fluid communication with the vacuum pump provided in the controller/actuation module 203 and is also in fluid communication with the holes forming the apertures 206 of the outer tip member 222. Fluid communication between the internal cavity 214 and the vacuum pump is provided via an internal conduit 226 running through the proximal portion 220b of the support member 220. The internal conduit 226 fluidly connects the internal cavity 214 and the flexible lead 204.

The support member 220 further comprises an arrangement of passageways 228 that fluidly connect the internal cavity 214 with the holes of the outer tip member 222 which form the apertures 206. **In** the present embodiment, the passageways 228 are formed by a combination of holes 230 extending through the wall of the support member (which open into the internal cavity 214) and channels formed on the outer surface of the support member. When the outer tip member 222 is fit over the support member 220 its inner surface is spaced apart from parts of the outer surface of the support member to define the channels therebetween. **In** the present embodiment, the channels are formed by ridges 232 that define circumferential channels 230a. Cut-outs 234 in the ridges define axial channels to interconnect the circumferential channels along the length of the device so that each circumferential channel does not require a corresponding hole 230. Other structural arrangements may be provided to form the passage ways within the support member 220. For example, the cut-outs may be absent, with a hole 230 provided for each circumferential channel.

The support member 220 is arranged to interlock with the outer tip member so that the holes 206 of the outer tip member are orientated relative to the passageways of the support member 220 and a fluid connection is provided between them. A keyed interlocking arrangement is provided to ensure alignment and fluidic connection. **In** the present embodiment, the holes of the outer tip member 222 are aligned with the channels formed on the outer surface of the support member so that they are fluidly connected. This allows easy assembly of the components of the device.

The treatment module 201 further comprises a liquid trap 236 arranged to trap liquid (e.g. water) discharge from the vagina that may be drawn through the apertures during use. The liquid trap 236 is provided at a position upstream of the pump (along the fluidic connection path between the pump and the apertures 206) so as to trap any liquid drawn through the apertures 206 before it reaches the pump. In the present embodiment, the liquid tap 236 is located within the handle portion 207 of the support member 220 so that it is within the fluid communication path between the cavity 214 and the flexible lead 204 (i.e. it is located at a point along the conduit 226). In other embodiments, the liquid trap 236 may be mounted elsewhere within the treatment module 201, or provided at the controller actuation module 203 or within the lead 204. In yet other embodiments it may be absent.

In various embodiments, the VA device 200 may comprise a hydrophobic filter. The hydrophobic filter may be provided instead of the liquid trap. The hydrophobic filter is arranged to block contaminates drawn though the apertures 206 from reaching the vacuum pump. The hydrophobic filter comprises a filter module having a hydrophobic filter membrane (e.g. a PTFE membrane). The filter module may be provided in the treatment module, and may be arranged to block contaminates from passing along flexible lead or tubing connecting the treatment module to the pump. The filter module may alternatively be provided at any point on the fluid connection path upstream of the pump, e.g. at the connection point between the flexible lead and the actuator/controller module.

Referring to Figure 24, the actuation/controller module 203 is shown in more detail. The actuation/controller module 203 comprises a housing formed by a first (or top) part 250 and a second (or bottom) part 252. The first and second parts of the housing are connected by connecting means 254 in the form of bolts. In other embodiments, other connecting means such as adhesive may be used. The controller actuation module 203 further comprises a vacuum pump 256 mounted within the housing, the vacuum pump being fluidly connected to the flexible lead 204 via tubing 258. The vacuum pump is arranged to provide the vacuum pressure at the apertures as described above. The tubing 258 comprises a connector 260 in the form of a T-connector. The T-connector is arranged to split the output of the pump into a first output which connects to the treatment module (e.g. via the flexible lead 204) and a second output which is connected to a sensor provided within the actuation/controller module (as shown schematically in Figure 2, with the sensor provided at the controller 11). The second output and sensor provide a feedback loop to monitor pressure during use, and allow the operation of the pump to be adjusted accordingly. The actuation/controller module 203 further comprises a microcontroller 262 in operative communication with the pump 256 to provide control of the therapy delivery. The microcontroller 262 is further in operative communication with a user interface comprising a main control button 264 and an LED panel 266. The actuation/controller module further comprises a battery 268 and a power port 270 to provide electrical power. Various connectors 272, 274, 276, 278 are provided to mount components within the housing. The connectors may be in the form of bolts as shown, although other types of component or connecting means may be provided.

### Experiment test results

A series of experimental tests have been conducted with both ex vivo tissue and in vivo to demonstrate the safety and efficacy of the VA treatment. Preclinical testing of a device according to the embodiments shown in Figures 14, 15 and 16 in an ovine model has shown that the microtrauma process and subsequent healing results in thickening of the vaginal wall, angiogenesis (i.e. blood vessel growth) and enhanced capillary flow. Small vaginal hematomas (pin-prick bleeds) have been found not to cause any adverse bleeding or haemorrhages as the mechanism of action causes a "microtrauma" only to the small blood vessels.

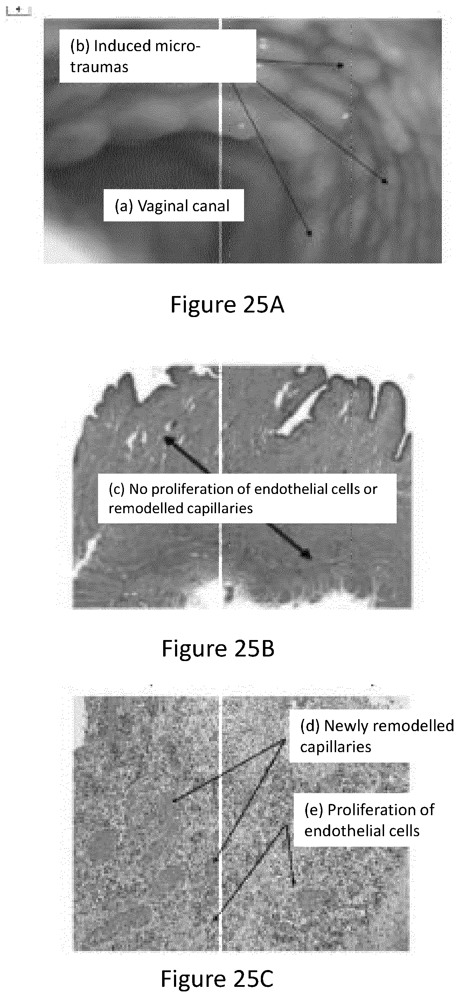

Figure 25a shows the vaginal wall (labelled "(a)") after therapy delivery using a device of the type illustrated in Figures 14, 15 and 16 as described above. Figure 25a shows an array of microtraumas (labelled "(b)") that were induced in the ovine module by drawing tissue into the apertures under application of negative pressure. Figure 25b shows a histology control image demonstrating that the vagina tissue was not exposed to an inflammation induced angiogenesis (label "(c)" indicates no proliferation of endothelial cells or remodelled capillaries). Figure 25c shows an image obtained using histology showing angiogenesis and healing effect in the vagina wall 3 weeks post treatment. Label "(d)" indicates newly remodelled capillaries and "(e)" proliferation of endothelial cells.

Various modifications will be apparent to the skilled person without departing from the scope of the claims.

### Equivalents

The foregoing description details presently preferred embodiments of the present invention. Numerous modifications and variations in practice thereof are expected to occur to those skilled in the art upon consideration of these descriptions.

## Claims

1. A vaginal atrophy treatment device (1, 20, 100, 200) comprising a treatment module (2, 21, 101, 201) configured for insertion into the vagina, at least part of the treatment module having an elongate, atraumatic shape and an outer surface configured to abut the wall of the vagina when inserted, the treatment module having a microtrauma module configured to deliver negative pressure mechanical microtrauma therapy to a wall of the vagina when inserted, wherein:
the microtrauma module comprises a plurality of apertures (6, 106, 206) formed by holes extending from the outer surface of the treatment module configured to abut the wall of the vagina when inserted,
the holes have a maximum cross-sectional size of greater than 1.0 mm and less than 4 mm,
the apertures being arrayed over at least part of the outer surface of the treatment module and the microtrauma module (2, 21, 101) is configured to deliver the negative pressure therapy to the wall of the vagina in contact with the apertures,
the vaginal atrophy treatment device further comprises a vacuum pump and the apertures are in fluidic connection with the vacuum pump,
the device is arranged to generate, in use, a negative pressure in the range between 200 mmHg and 600 mmHg at the apertures (6, 106), and
the device comprises a timer configured to switch off the device after a pre-defined treatment period, wherein the duration of the treatment period is between 1 and 90 minutes.

2. A device according to Claim 1, in which the device is arranged to generate a pressure in the range between 300 mmHg and 450 mmHg, at the apertures.

3. A device according to Claim 1 or Claim 2, in which the holes extend: a depth of at least 1 mm, and preferably less than 6 mm; or a depth of greater than 2 mm, and preferably less than 6 mm.

4. A device according to Claim 3, in which the holes extend a depth in the range between 3 mm and 4 mm, and even more preferably a depth of 3.5 mm.

5. A device according to any preceding Claim, in which the holes have a maximum cross-sectional size in the range between 2 mm and 3 mm, and even more preferably of 2.5 mm.

6. A device according to any preceding Claim, in which a portion of the treatment module adapted for insertion into the vagina has a maximum cross-sectional size in the range between 20 mm and 30 mm.

7. A device according to any preceding Claim, in which the treatment module (2, 21, 101, 201) comprises:
a support member (220), the support member (220) comprising an arrangement of passageways (228) that are fluidly coupled to the vacuum pump;
an outer tip member (222), the outer tip member (222) comprising a plurality of holes forming the apertures (206) of the treatment module,
wherein the outer tip member (222) is arranged to fit around a distal portion of the support member (222), the outer tip member (222) and support member (220) arranged to form an interlocking engagement whereby the passageways of the support member (220) are aligned relative to the holes of the outer tip member (222) to form a fluidic connection between them, and optionally:
one or both of:
a) the outer tip member (222) is formed from an elastomeric material, and the support member (220) is formed from a relatively rigid material compared to the outer tip member (222); and
b) a friction fit is provided between the support member (220) and the outer tip member (222).

8. A device according to any preceding Claim, further comprising:
a) a liquid trap (236), the liquid trap (236) being provided upstream of the vacuum pump and arranged to trap liquid discharge drawn through the apertures; and/or
b) a hydrophobic filter, the hydrophobic filter being arranged to block contaminates drawn though the apertures from reaching the vacuum pump.

9. A device according to any preceding Claim, in which the treatment module comprises a proximal handle part (7) and optionally:
a) in which the proximal portion of the treatment part (105) is a proximal sealing portion (105a) in which none of the apertures are located; and/or
b) in which the distal treatment part (21) is detachable from the proximal handle part (22), and in which the distal treatment part is disposable.

10. A device as claimed in any preceding Claim, in which the device comprises a remote actuation/controller module (3) operatively connectable to the treatment module (2) and comprising actuating and/or controlling means including the vacuum pump for the microtrauma module.

11. A device according to Claim 10, in which the treatment module (2) is detachable from the remote actuation/controller module (3) and is disposable, and optionally in which the actuation/controller module (3) comprises a graphical user interface.

12. A device according to Claim 11, in which the treatment module (2) and the actuation/controller (3) are coupled by a flexible lead (4), wherein: the flexible lead is detachable from the actuation/controller module so that the flexible lead (4) and the treatment module (2) are detachable from the actuation/controller module (3) and are disposable parts; or wherein treatment module (2) is detachable from the flexible lead (4) so that the treatment module (2) is a disposable part; and optionally:
the device further comprises a tamper resistance device (4a), provided at a connection point between disposable and non-disposable parts of the device, and wherein the actuator/controller module (3) is arranged to:
sense the tamper resistance device and to determine if the disposable parts are being used for the first time based on the tamper resistance device, and
prevent activation of the vacuum pump if disposable parts are determined to have been reused, and further optionally wherein:
a) the tamper resistance device comprises a unique identifier, and the device further comprises a sensor arranged to read the unique identifier, the actuation/controller module (3) being arranged to determine if the flexible lead (4) is being connected for the first time based on the unique identifier; or
b) the tamper resistance device is arranged to be altered or removed during connection of the flexible lead (4) for the first time, and the device further comprises a sensor arranged to sense the presence or condition of the tamper resistance device, the actuation/controller module (3) being arranged to determine whether the flexible lead (4) is being connected for the first time based on the presence or condition of the tamper resistance device.

13. A device according to any preceding Claim, in which any one or more of:
a) the treatment module comprises a moisture sensor;
b) the plurality of small apertures (6) are arranged circumferentially around the microtrauma module, or in which the plurality of small apertures (6) are arranged bilaterally in discrete arrays on the microtrauma module;
c) the device comprises a pressure sensor (10) configured to determine an operational pressure of the negative pressure therapy being applied to the wall of the vagina, and a processor (11) configured to receive data relating to the operational pressure from the pressure sensor, compare the detected operational pressure with a reference pressure, and modify the operational pressure to achieve a target pressure when a sub-optimal operational pressure is detected;
d) the device comprises a camera and optionally an illumination system configured for imaging the wall of the vagina during use; and/or
e) the device is configured to relay data to a mobile user device and to receive control data from the mobile user device for control of the therapy.

## Patentansprüche

1. Vorrichtung (1, 20, 100, 200) zur Behandlung vaginaler Atrophie, umfassend ein Behandlungsmodul (2, 21, 101, 201), das zur Einführung in die Vagina konfiguriert ist, wobei mindestens ein Teil des Behandlungsmoduls eine längliche, atraumatische Form und eine Außenfläche aufweist, die dazu konfiguriert ist, an der Wand der Vagina anzuliegen, wenn es eingeführt wird, wobei das Behandlungsmodul ein Mikrotraumamodul aufweist, das dazu konfiguriert ist, eine mechanische Mikrotrauma-Unterdrucktherapie an eine Wand der Vagina abzugeben, wenn es eingeführt wird, wobei:
das Mikrotraumamodul eine Vielzahl von Öffnungen (6, 106, 206) umfasst, die durch Löcher gebildet sind, die sich von der Außenfläche des Behandlungsmoduls erstrecken, die dazu konfiguriert ist, an der Wand der Vagina anzuliegen, wenn es eingeführt wird,
die Löcher eine maximale Querschnittsgröße von mehr als 1,0 mm und weniger als 4 mm aufweisen,
die Öffnungen über mindestens einen Teil der Außenfläche des Behandlungsmoduls verteilt sind und das Mikrotraumamodul (2, 21, 101) dazu konfiguriert ist, die Unterdrucktherapie an die Wand der Vagina, welche die Öffnungen berührt, abzugeben,
die Vorrichtung zur Behandlung vaginaler Atrophie ferner eine Vakuumpumpe umfasst und die Öffnungen in Fluidverbindung mit der Vakuumpumpe stehen,
die Vorrichtung dazu ausgelegt ist, bei Verwendung einen Unterdruck in dem Bereich zwischen 200 mmHg und 600 mmHg an den Öffnungen (6, 106) zu erzeugen, und
die Vorrichtung einen Zeitgeber umfasst, der dazu konfiguriert ist, die Vorrichtung nach einem vordefinierten Behandlungszeitraum abzuschalten, wobei die Dauer des Behandlungszeitraums zwischen 1 und 90 Minuten liegt.

2. Vorrichtung nach Anspruch 1, wobei die Vorrichtung dazu ausgelegt ist, einen Druck in dem Bereich zwischen 300 mmHg und 450 mmHg an den Öffnungen zu erzeugen.

3. Vorrichtung nach Anspruch 1 oder Anspruch 2, wobei sich die Löcher wie folgt erstrecken: in eine Tiefe von mindestens 1 mm und vorzugsweise weniger als 6 mm; oder in eine Tiefe von mehr als 2 mm und vorzugsweise weniger als 6 mm.

4. Vorrichtung nach Anspruch 3, wobei sich die Löcher in eine Tiefe in dem Bereich zwischen 3 mm und 4 mm und noch mehr bevorzugt in eine Tiefe von 3,5 mm erstrecken.

5. Vorrichtung nach einem der vorhergehenden Ansprüche, wobei die Löcher eine maximale Querschnittsgröße in dem Bereich zwischen 2 mm und 3 mm und noch mehr bevorzugt von 2,5 mm aufweisen.

6. Vorrichtung nach einem der vorhergehenden Ansprüche, wobei ein Abschnitt des Behandlungsmoduls, der zur Einführung in die Vagina ausgelegt ist, eine maximale Querschnittsgröße in dem Bereich zwischen 20 mm und 30 mm aufweist.

7. Vorrichtung nach einem der vorhergehenden Ansprüche, wobei das Behandlungsmodul (2, 21, 101, 201) Folgendes umfasst:
ein Stützelement (220), wobei das Stützelement (220) eine Anordnung von Durchgängen (228) umfasst, die fluidisch mit der Vakuumpumpe gekoppelt sind;
ein äußeres Spitzenelement (222), wobei das äußere Spitzenelement (222) eine Vielzahl von Löchern umfasst, welche die Öffnungen (206) des Behandlungsmoduls bilden,
wobei das äußere Spitzenelement (222) dazu ausgelegt ist, dass es um einen distalen Abschnitt des Stützelements (222) passt, das äußere Spitzenelement (222) und das Stützelement (220) dazu ausgelegt sind, dass sie einen Verriegelungseingriff bilden, wodurch die Durchgänge des Stützelements (220) relativ zu den Löchern des äußeren Spitzenelements (222) ausgerichtet sind, um eine Fluidverbindung zwischen ihnen zu bilden, und optional:
wobei eines oder beides von Folgendem zutrifft:
a) das äußere Spitzenelement (222) ist aus einem elastomeren Material gebildet und das Stützelement (220) ist im Vergleich zu dem äußeren Spitzenelement (222) aus einem relativ starren Material gebildet; und
b) ein Reibschluss ist zwischen dem Stützelement (220) und dem äußeren Spitzenelement (222) bereitgestellt.

8. Vorrichtung nach einem der vorhergehenden Ansprüche, ferner umfassend:
a) einen Flüssigkeitsauffangbehälter (236), wobei der Flüssigkeitsauffangbehälter (236) vor der Vakuumpumpe bereitgestellt und dazu ausgelegt ist, eine Flüssigkeitsableitung, die durch die Öffnungen gesaugt wird, aufzufangen; und/oder
b) ein hydrophobes Filter, wobei das hydrophobe Filter dazu ausgelegt ist, zu verhindern, dass Verunreinigungen, die durch die Öffnungen gesaugt werden, die Vakuumpumpe erreichen.

9. Vorrichtung nach einem der vorhergehenden Ansprüche, wobei das Behandlungsmodul einen proximalen Griffteil (7) umfasst und optional:
a) wobei der proximale Abschnitt des Behandlungsteils (105) ein proximaler Dichtungsabschnitt (105a) ist, in dem sich keine der Öffnungen befindet; und/oder
b) wobei der distale Behandlungsteil (21) von dem proximalen Griffteil (22) abnehmbar ist und wobei der distale Behandlungsteil wegwerfbar ist.

10. Vorrichtung nach einem der vorhergehenden Ansprüche, wobei die Vorrichtung ein Fernbetätigungs-/Fernsteuerungsmodul (3) umfasst, das mit dem Behandlungsmodul (2) wirkverbindbar ist und Betätigungs-und/oder Steuerungsmittel umfasst, welche die Vakuumpumpe für das Mikrotraumamodul beinhalten.

11. Vorrichtung nach Anspruch 10, wobei das Behandlungsmodul (2) von dem Fernbetätigungs-/Fernsteuerungsmodul (3) abnehmbar und wegwerfbar ist und optional wobei das Betätigungs-/Steuerungsmodul (3) eine grafische Benutzerschnittstelle umfasst.

12. Vorrichtung nach Anspruch 11, wobei das Behandlungsmodul (2) und die Betätigung/Steuerung (3) durch ein flexibles Kabel (4) gekoppelt sind, wobei: das flexible Kabel von dem Betätigungs-/Steuerungsmodul abnehmbar ist, sodass das flexible Kabel (4) und das Behandlungsmodul (2) von dem Betätigungs-/Steuerungsmodul (3) abnehmbar sind und wegwerfbare Teile sind; oder wobei das Behandlungsmodul (2) von dem flexiblen Kabel (4) abnehmbar ist, sodass das Behandlungsmodul (2) ein wegwerfbares Teil ist; und optional:
wobei die Vorrichtung ferner eine Manipulationssicherheitsvorrichtung (4a) umfasst, die an einem Verbindungspunkt zwischen wegwerfbaren und nicht wegwerfbaren Teilen der Vorrichtung bereitgestellt ist, und wobei das Betätigungs-/Steuerungsmodul (3) zu Folgendem ausgelegt ist:
Erfassen der Manipulationssicherheitsvorrichtung und Bestimmen, ob die wegwerfbaren Teile zum ersten Mal verwendet werden, basierend auf der Manipulationssicherheitsvorrichtung, und
Verhindern einer Aktivierung der Vakuumpumpe, wenn bestimmt wird, dass wegwerfbare Teile wiederverwendet wurden, und ferner optional wobei:
a) die Manipulationssicherheitsvorrichtung eine eindeutige Kennung umfasst und die Vorrichtung ferner einen Sensor umfasst, der dazu ausgelegt ist, die eindeutige Kennung zu lesen, wobei das Betätigungs-/Steuerungsmodul (3) dazu ausgelegt ist, zu bestimmen, ob das flexible Kabel (4) zum ersten Mal verbunden wird, basierend auf der eindeutigen Kennung; oder
b) die Manipulationssicherheitsvorrichtung dazu ausgelegt ist, während der Verbindung des flexiblen Kabels (4) zum ersten Mal verändert oder entfernt zu werden, und die Vorrichtung ferner einen Sensor umfasst, der dazu ausgelegt ist, das Vorhandensein oder den Zustand der Manipulationssicherheitsvorrichtung zu erfassen, wobei das Betätigungs-/Steuerungsmodul (3) dazu ausgelegt ist, zu bestimmen, ob das flexible Kabel (4) zum ersten Mal verbunden wird, basierend auf dem Vorhandensein oder dem Zustand der Manipulationssicherheitsvorrichtung.

13. Vorrichtung nach einem der vorhergehenden Ansprüche, wobei eines oder mehrere von Folgendem zutreffen:
a) das Behandlungsmodul umfasst einen Feuchtigkeitssensor;
b) die Vielzahl von kleinen Öffnungen (6) ist umlaufend um das Mikrotraumamodul angeordnet oder die Vielzahl von kleinen Öffnungen (6) ist bilateral in einzelnen Gruppen auf dem Mikrotraumamodul angeordnet;
c) die Vorrichtung umfasst einen Drucksensor (10), der dazu konfiguriert ist, einen Betriebsdruck der Unterdrucktherapie zu bestimmen, die auf die Wand der Vagina angewendet wird, und einen Prozessor (11), der dazu konfiguriert ist, Daten in Bezug auf den Betriebsdruck von dem Drucksensor zu empfangen, den erkannten Betriebsdruck mit einem Referenzdruck zu vergleichen und den Betriebsdruck zu modifizieren, um einen Solldruck zu erreichen, wenn ein suboptimaler Betriebsdruck erkannt wird;
d) die Vorrichtung umfasst eine Kamera und optional ein Beleuchtungssystem, die dazu konfiguriert sind, die Wand der Vagina während der Verwendung abzubilden; und/oder
e) die Vorrichtung ist dazu konfiguriert, Daten an eine mobile Benutzervorrichtung weiterzuleiten und Steuerungsdaten von der mobilen Benutzervorrichtung zur Steuerung der Therapie zu empfangen.

## Revendications

1. Dispositif de traitement de l'atrophie vaginale (1, 20, 100, 200) comprenant un module de traitement (2, 21, 101, 201) configuré pour une insertion dans le vagin, au moins une partie du module de traitement ayant une forme allongée, atraumatique et une surface externe configurée pour venir en butée contre la paroi du vagin lors de l'insertion, le module de traitement ayant un module de microtraumatismes configuré pour administrer une thérapie par microtraumatismes mécaniques par pression négative à une paroi du vagin lors de l'insertion, dans lequel :
le module de microtraumatismes comprend une pluralité d'ouvertures (6, 106, 206) formées par des trous s'étendant à partir de la surface externe du module de traitement configurée pour venir en butée contre la paroi du vagin lors de l'insertion,
les trous ont une dimension maximale en section transversale supérieure à 1,0 mm et inférieure à 4 mm,
les ouvertures étant disposées en réseau sur au moins une partie de la surface externe du module de traitement et le module de microtraumatismes (2, 21, 101) est configuré pour administrer la thérapie par pression négative à la paroi du vagin en contact avec les ouvertures,
le dispositif de traitement de l'atrophie vaginale comprend en outre une pompe à vide et les ouvertures sont en connexion fluidique avec la pompe à vide,
le dispositif est agencé pour générer, en utilisation, une pression négative dans la plage entre 200 mmHg et 600 mmHg au niveau des ouvertures (6, 106), et
le dispositif comprend une minuterie configurée pour éteindre le dispositif après une période de traitement prédéfinie, dans lequel la durée de la période de traitement est entre 1 et 90 minutes.

2. Dispositif selon la revendication 1, dans lequel le dispositif est agencé pour générer une pression dans la plage entre 300 mmHg et 450 mmHg, au niveau des ouvertures.

3. Dispositif selon la revendication 1 ou la revendication 2, dans lequel les trous s'étendent sur : une profondeur d'au moins 1 mm, et de préférence inférieure à 6 mm ; ou une profondeur supérieure à 2 mm, et de préférence inférieure à 6 mm.

4. Dispositif selon la revendication 3, dans lequel les trous s'étendent sur une profondeur dans la plage entre 3 mm et 4 mm, et encore plus de préférence une profondeur de 3,5 mm.

5. Dispositif selon l'une quelconque des revendications précédentes, dans lequel les trous ont une dimension maximale en section transversale dans la plage entre 2 mm et 3 mm, et encore plus de préférence de 2,5 mm.

6. Dispositif selon l'une quelconque des revendications précédentes, dans lequel une partie du module de traitement adaptée pour l'insertion dans le vagin a une dimension maximale en section transversale dans la plage entre 20 mm et 30 mm.

7. Dispositif selon l'une quelconque des revendications précédentes, dans lequel le module de traitement (2, 21, 101, 201) comprend :
un élément de support (220), l'élément de support (220) comprenant un agencement de voies de passage (228) qui sont couplées de manière fluide à la pompe à vide ;
un élément d'embout externe (222), l'élément d'embout externe (222) comprenant une pluralité de trous formant les ouvertures (206) du module de traitement,
dans lequel l'élément d'embout externe (222) est agencé pour s'ajuster autour d'une partie distale de l'élément de support (222), l'élément d'embout externe (222) et l'élément de support (220) étant agencés pour former un engagement par emboîtement par lequel les voies de passage de l'élément de support (220) sont alignées par rapport aux trous de l'élément d'embout externe (222) pour former une connexion fluidique entre eux, et optionnellement :
l'un ou les deux parmi :
a) l'élément d'embout externe (222) est formé à partir d'un matériau élastomère, et l'élément de support (220) est formé à partir d'un matériau relativement rigide par rapport à l'élément d'embout externe (222) ; et
b) un ajustement par friction est prévu entre l'élément de support (220) et l'élément d'embout externe (222).

8. Dispositif selon l'une quelconque des revendications précédentes, comprenant en outre :
a) un piège à liquide (236), le piège à liquide (236) étant prévu en amont de la pompe à vide et agencé pour piéger les évacuations de liquide aspirées à travers les ouvertures ; et/ou
b) un filtre hydrophobe, le filtre hydrophobe étant agencé pour bloquer les contaminants aspirés à travers les ouvertures pour les empêcher d'atteindre la pompe à vide.

9. Dispositif selon l'une quelconque des revendications précédentes, dans lequel le module de traitement comprend une partie de poignée proximale (7) et optionnellement : a) dans lequel la partie proximale de la partie de traitement (105) est une partie d'étanchéité proximale (105a) dans laquelle aucune des ouvertures n'est située ; et/ou b) dans lequel la partie de traitement distale (21) est détachable de la partie de poignée proximale (22), et dans lequel la partie de traitement distale est jetable.

10. Dispositif tel que revendiqué dans l'une quelconque des revendications précédentes, dans lequel le dispositif comprend un module actionneur/dispositif de commande à distance (3) connectable de manière opérationnelle au module de traitement (2) et comprenant des moyens d'actionnement et/ou de commande incluant la pompe à vide pour le module de microtraumatismes.

11. Dispositif selon la revendication 10, dans lequel le module de traitement (2) est détachable du module actionneur/dispositif de commande à distance (3) et est jetable, et optionnellement dans lequel le module actionneur/dispositif de commande (3) comprend une interface graphique utilisateur.

12. Dispositif selon la revendication 11, dans lequel le module de traitement (2) et le module actionneur/dispositif de commande (3) sont couplés par un fil conducteur flexible (4), dans lequel : le fil conducteur flexible est détachable du module actionneur/dispositif de commande de sorte que le fil conducteur flexible (4) et le module de traitement (2) soient détachables du module actionneur/dispositif de commande (3) et soient des pièces jetables ; ou dans lequel le module de traitement (2) est détachable du fil conducteur flexible (4) de sorte que le module de traitement (2) soit une pièce jetable ; et optionnellement :
le dispositif comprend en outre un dispositif d'inviolabilité (4a), prévu au niveau d'un point de connexion entre des pièces jetables et non jetables du dispositif, et dans lequel le module actionneur/dispositif de commande (3) est agencé pour :
détecter le dispositif d'inviolabilité et pour déterminer si les pièces jetables sont utilisées pour la première fois sur la base du dispositif d'inviolabilité, et
empêcher l'activation de la pompe à vide s'il est déterminé que des pièces jetables ont été réutilisées, et en outre optionnellement dans lequel :
a) le dispositif d'inviolabilité comprend un identifiant unique, et le dispositif comprend en outre un capteur agencé pour lire l'identifiant unique, le module actionneur/dispositif de commande (3) étant agencé pour déterminer si le fil conducteur flexible (4) est connecté pour la première fois sur la base de l'identifiant unique ; ou
b) le dispositif d'inviolabilité est agencé pour être altéré ou retiré lors de la connexion du fil conducteur flexible (4) pour la première fois, et le dispositif comprend en outre un capteur agencé pour détecter la présence ou l'état du dispositif d'inviolabilité, le module actionneur/dispositif de commande (3) étant agencé pour déterminer si le fil conducteur flexible (4) est connecté pour la première fois sur la base de la présence ou de l'état du dispositif d'inviolabilité.

13. Dispositif selon l'une quelconque des revendications précédentes, dans lequel l'une ou plusieurs quelconques parmi :
a) le module de traitement comprend un capteur d'humidité ;
b) la pluralité de petites ouvertures (6) sont agencées de manière circonférentielle autour du module de microtraumatismes, ou dans lequel la pluralité de petites ouvertures (6) sont agencées de manière bilatérale en réseaux distincts sur le module de microtraumatismes ;
c) le dispositif comprend un capteur de pression (10) configuré pour déterminer une pression opérationnelle de la thérapie par pression négative étant appliquée à la paroi du vagin, et un processeur (11) configuré pour recevoir des données relatives à la pression opérationnelle provenant du capteur de pression, comparer la pression opérationnelle détectée avec une pression de référence, et modifier la pression opérationnelle pour atteindre une pression cible lorsqu'une pression opérationnelle sous-optimale est détectée ;
d) le dispositif comprend une caméra et optionnellement un système d'éclairage configuré pour l'imagerie de la paroi du vagin lors de l'utilisation ; et/ou
e) le dispositif est configuré pour relayer des données vers un dispositif utilisateur mobile et pour recevoir des données de commande provenant du dispositif utilisateur mobile pour la commande de la thérapie.
